# EUROPEAN PATENT APPLICATION

(11) **EP 4 249 588 A1**
(43) Date of publication of application: **27.09.2023**
(21) Application number: 22163501.4
(22) Date of filing: 22.03.2022
(51) Int. Cl.: C12N 5/0783, A61K 35/12, C07K 14/47

(54) **CELLULAR THERAPY**

(71) Applicant: Universität Basel Vizerektorat Forschung, 4001 Basel (CH)
(72) Inventor: HESS, Christoph, 4051 Basel (CH); LOELIGER, Jordan, 4056 Basel (CH)
(74) Representative: Cooley (UK) LLP

(57) **Abstract**

The invention is in the field of regenerative medicine and provides compositions and methods for treating cancer and/or infections in patients. The invention provides cells, preferably immune cells, genetically engineered to enforce expression of PHGDH, and expression constructs, vectors and methods for preparing and using the same.

## Description

### Field

The present invention is in the field of regenerative medicine, in particular cellular therapy and provides compositions and methods for treating cancer and/or infections in patients. The invention provides cells, preferably immune cells, genetically engineered to enforce expression of PHGDH, and expression constructs, vectors and methods for preparing and using the same.

### Sequence listing

A sequence listing (entitled HORN-002_F01EP - Sequence Listing_ST25) associated with this application is provided electronically in text format in lieu of a paper copy and is hereby incorporated by reference.

### Background

Cellular therapy is the transfer of intact, live cells into a patient to treat a disease or condition. The cells may originate from the patient (autologous cells) or a donor (allogeneic cells), and recent development have enabled the use of cells from different organisms (xenogeneic).

Adoptive immune cell therapies (also referred to as cellular immunotherapies) are a form of cellular treatment that use the cells of our immune system to treat a disease or condition.

Our immune system is capable of recognizing and eliminating cells that have become infected or damaged as well as those that have become cancerous. In the case of cancer, immune cells known as cytotoxic T cells (CTLs or CD8⁺ T cells) are particularly powerful due to their ability to bind to suitable cell-surface molecules on cancer cells known as antigens.

CD8⁺ T cells are critically involved in protecting the host from viruses, intracellular bacteria and malignant cells. During acute infection, naive CD8⁺ T (T_{NV}) cells are primed in lymph nodes, expand clonally, and differentiate into primary effector cells. Effector cells then leave the lymph node, enter the bloodstream and migrate to sites of inflammation, where they secrete cytokines and kill infected cells. Once the pathogen is cleared, the majority of effector cells undergoes apoptosis during contraction phase, whereas a small proportion of antigen-specific CD8⁺ T cells differentiates to form the memory pool. A defining feature of memory T cells is their capacity to re-expand and acquire effector function upon encountering the same antigen (secondary effector cells) more rapidly than their naive counterparts - enabling increased efficacy to contain re-infection. Reflecting the organization of the recall response, various subsets of memory T cells exist that differ with regard to migration pattern and tissue distribution, phenotype and functional properties. Human central memory (T_{CM}) and effector memory (T_{EM}) CD8⁺ T cells are readily accessible in peripheral blood. The former re-circulate between the blood stream and lymph nodes, the latter home from the blood circulation into distinct peripheral tissue sites.

CD4⁺ T cells exhibit a diverse repertoire of effector functions and exhibit considerable phenotypic plasticity and heterogeneity depending on local context and microenvironment. A key role of CD4⁺ T cells is to modulate the state and function of other immune cells. By the virtue of their inherent ability to orchestrate a wide range of immune responses and their flexible differentiation into different effector lineages, CD4⁺ T cells are critical for an optimal anti-tumor response, including cancer immunotherapy. Effector CD4⁺ T cells contribute to anti-tumor responses through different major strategies, such as exertion of direct anti-tumor activity by producing IFN-y and TNF-α. Activated CD4⁺ T cells secrete IL-2, which directly activates CD8⁺ CTLs by driving their effector function, differentiation and proliferation. CD4⁺ T cells provide indirect help to CD8⁺ T cells by supporting and maintaining pro-inflammatory cross-presenting dendritic cells (DCs), which in turn activate CD8⁺ T cells. CD4⁺ T cells are also indispensable for the induction of humoral responses against tumour antigens by orchestrating antibody production.

Cancer patients may have endogenous T cells that are capable of targeting their cancer cells. CD8⁺ T cells in particular are capable of recognizing and eliminating cancer cells in a very precise way. However, the presence of these cells doesn't necessarily mean that they are sufficient to clear the tumor. The cells must also be able to maintain that activity, in sufficient numbers and for a sufficiently long time to sustain an effective anti-tumor response.

Some adoptive cellular therapy approaches involve directly isolating our own immune cells and expanding their numbers before reinfusion (e.g. TILs), whereas other approaches involve genetically engineering the immune cells to enhance their effector functions (e.g. CAR T cells). In particular, chimeric antigen receptor (CAR) T cell approaches are emerging as strategies with the highest potential for the treatment of disease, in particular cancer, such as various malignancies.

CAR T cell therapy, relies on redirecting T cells to a suitable cell-surface molecule on cancer cells and shows promising results in harnessing the power of the immune system to treat malignancies and other cancers. Gamma-retroviral and lentiviral transduction approaches are most often used in genetically engineered CAR T cells due to their high transduction efficiencies. Most currently available CAR-T therapies are manufactured using lentiviruses. Therapies using CAR T cells have shown very promising results in various tumor types, especially in the treatment of hematologic malignancies. In 2017, the two first CAR T cell therapies, Kymriah (tisagenlecleucel, Novartis) and Yescarta (axicabtagene ciloleucel, Gilead), gained FDA approval. Both therapies are approved for patients with specific hematologic cancers refractive to standard therapies or relapsing disease. Such patients were previously considered incurable and left with virtually no treatment option.

Natural Killer (NK) Cell Therapy is another form of adoptive cellular therapy. These NK cells can also be equipped with cancer-targeting CARs.

Tumor-infiltrating lymphocyte (TIL) therapy is an adoptive cellular therapy approach that harvests naturally occurring T cells that have already infiltrated patients' tumors. The cells are activated and expanded *ex vivo* and then re-infused into patients.

Engineered T cell receptor (TCR) therapy is another adoptive cellular therapy approach that involves taking T cells from patients and equipping them with a new T cell receptor that targets specific cancer antigens, allowing doctors to choose an optimal target for each patient's tumor and distinct types of T cell to engineer.

Cellular therapies have also been investigated for the treatment of infectious diseases, in particular viral diseases such as chronic hepatitis B, CMV infection, EBV and HIV or opportunistic fungal infections. Viral infections or relapses developing in immunosuppressed patients (e.g., patients after hematopoietic stem cell or organ transplantation with human cytomegalovirus or EBV reactivation) have been shown to be responsive to adoptive immunotherapy with autologous *in vitro* expanded virus-specific T cells derived from memory or naïve T cell populations (Houghtelin A. et al. 2017, Front. Immunol. 8:1272. doi: 10.3389/fimmu.2017.01272).

Despite the great success of CAR T cells in treating certain hematologic malignancies, not all patients respond to adoptive cell therapy. A significant proportion of CAR T cell-treated patients experience a relapse and CAR T cell-based treatment of solid cancers remains a major obstacle. Besides the ability for CAR on genetically modifies T cells to recognize and destroy targeted cells, a successful therapeutic T cell therapy needs to have the ability to proliferate and persist over time, and to further monitor for tumor cell escapees. Therefore, engineering CAR T cells to improve their survival and thus persistence represents a logical therapeutic approach to enhance their therapeutic potential.

There remains a need in the art for improved cellular therapies, in particular improved adoptive cellular therapies such as CAR T cell therapies with improved survival and/or persistence. These cellular therapies would offer more effective treatments of diseases such as cancer and infections.

### Summary of the invention

In a first aspect, the invention provides a cell genetically engineered to enforce expression of phosphoglycerate dehydrogenase (PHGDH).

In a second aspect, the invention provides an expression cassette comprising a polynucleotide sequence encoding phosphoglycerate dehydrogenase (PHGDH).

In a third aspect, the invention provides a vector comprising a polynucleotide sequence encoding phosphoglycerate dehydrogenase (PHGDH), optionally wherein the polynucleotide sequence comprises SEQ ID NO: 4 or a sequence having 95% identity to SEQ ID NO: 4 and wherein the sequence encodes a polypeptide having RNA binding activity, or a variant or functional fragment thereof.

In a fourth aspect, the invention provides a cell comprising an expression cassette as described herein or a vector as described herein.

In a fifth aspect, the invention provides a composition comprising a cell as described herein, an expression cassette as described herein or a vector as described herein.

In a sixth aspect, the invention provides a method of treating a disease or condition in a subject in need thereof comprising administering to the subject a cell as described herein, an expression cassette as described herein, a vector as described herein or a composition as described herein.

In a seventh aspect, the invention provides a cell as described herein, an expression cassette as described herein, a vector as described herein or a composition as described herein, for use in a method of treating a disease or condition in a subject.

In an eight aspect, the invention provides a method of producing a cell genetically engineered to enforce expression of phosphoglycerate dehydrogenase (PHGDH) comprising contacting one or more cells with a vector as described herein.

### Brief description of the Figures

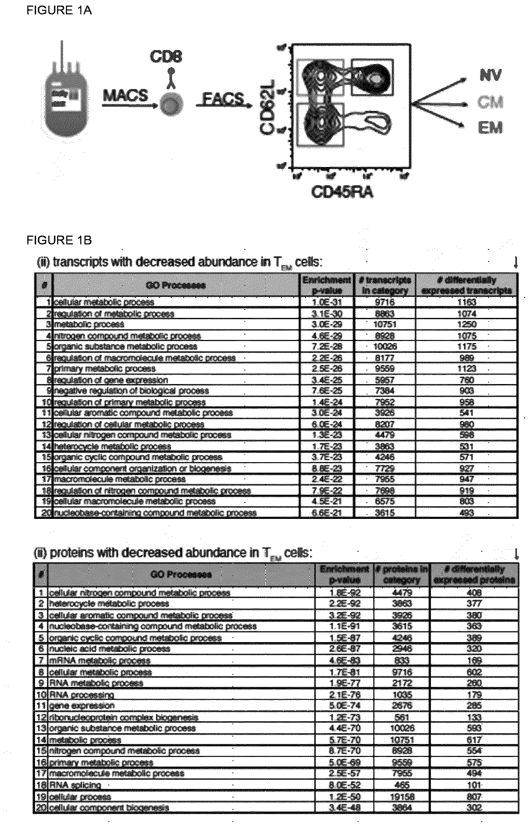

Figure 1A shows a schematic of T cell isolation and sorting strategy.
Figure 1B shows biological processes overrepresented among differentially expressed transcripts/proteins between freshly sorted human T_{NV} and T_{EM} cells based on RNAseq (upper *panel*) and proteomics data (*lower panel*). Specifically, GO term enrichment analysis was performed on transcripts/proteins with decreased abundance in T_{EM} cells compared to T_{NV} cells. From each analysis, the 20 biological processes with lowest p-values are shown in a ranked order. From MetaCore (Clarivate Analytics).

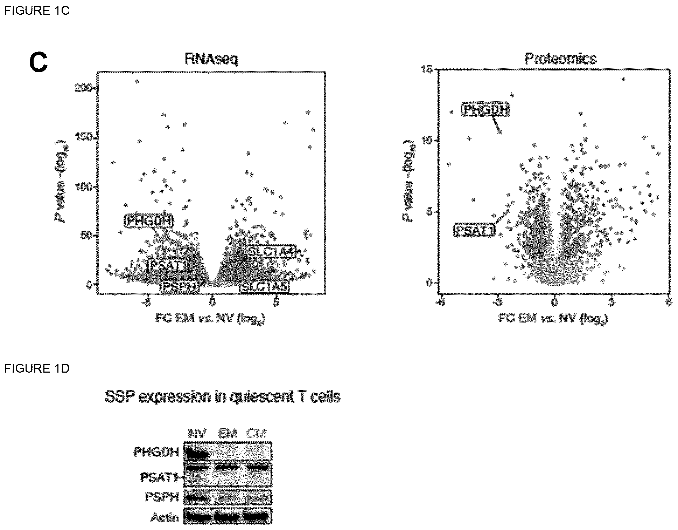

Figure 1C shows a volcano plot of RNAseq- *(left panel)* and proteomics (*right panel*) analysis comparing T_{NV} and T_{EM} cells from healthy human donors (n=4). Detected transcripts/proteins related to serine metabolism are shown in red. Hits with p_{adj}<0.05 are marked in dark blue if log₂FC>0.5 or turquoise if log₂FC<0.5.
Figure 1D shows a Western blot analyzing expression of enzymes of the SSP in freshly sorted T_{NV}, T_{EM} and T_{CM} cells. Data are representative of two independent experiments.

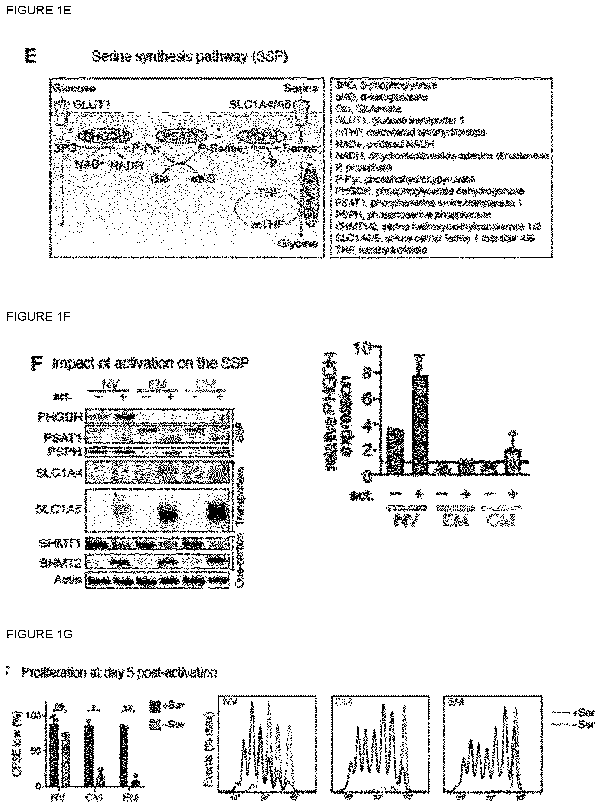

Figure 1E is a schematic illustration of the SSP enzymes (green), serine permeable amino acid transporters (yellow) and enzymes representing the connection to one carbon metabolism (blue).
Figure 1F is a representative Western blot analyzing expression of proteins involved in serine metabolism among T_{NV}, T_{EM} and T_{CM} cells. T cells were left unstimulated (-) or activated using an anti-CD3/CD28 mAb (+) coated beads for 24h *(left panel).* Quantification of PHGDH protein abundance by densitometry, normalized against actin and represented relative to PHGDH abundance in activated T_{EM} cells of each respective donor (3 independent experiments, *right panel).*
Figure 1G shows assessment of proliferation by CFSE dye dilution 5 days post-activation. Bar graph summary indicating the percentage of cells, which have diluted the dye at least by one cell division (n=3, *left panel*). Representative histogram of CFSE dilution of T_{NV}, T_{CM} and T_{EM} cells from one human donor (*right panel*). Data are represented as mean ±SD (F,G). P values were determined using a paired two-tailed Student's t-test. ^{∗}P<0.05, ^{∗∗}P<0.01; ns, not significant.

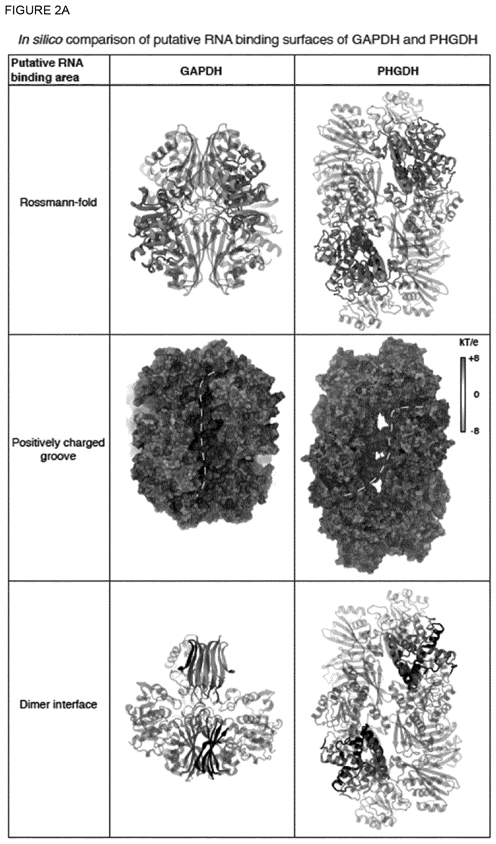

Figure 2A shows a comparison of putative RNA-binding sites in GAPDH (*left*) and PHGDH (*right*)*.* Structural features of GAPDH involved in RNA-binding (Ref. 5) are highlighted and compared to PHGDH. The Rossman fold domains of each two interacting subunits are shown in green (a-helices) and blue (β-sheets), whereas R163 is labeled in red (*top panel*).
Electrostatic potential of surface residues. The yellow lines highlight regions of high positive potentials, possibly involved in electrostatic interactions with negatively charged RNA (*middle panel*). Dimer interface. For GAPDH, the two ensembles of five stranded β-sheets are shown in black and orange, one color per subunit - the rest of the protein is shown in pale grey ribbons. In the interface shown, one subunit has been removed for clarity - except residues of the interface. For PHGDH, the dimer interface is shown in black and orange, one color per subunit - the rest of the protein is shown in pale grey ribbons with R163 labeled in red (*bottom panel*).
Figure 2B shows specificity of PHGDH-antibody (#66350, D8F30, Cell Signaling) used for RIPseq experiment, was tested on PHGDH^{WT} (WT) and PHGDH^{KO} (KO) Jurkat T cell lysates and total protein stain *(left panel).* IP for RIPseq experiment was performed on sorted T_{NV} 36h post-activation using PHGDH and isotype control antibody and verified by Western Blot (n=3, *right panel).* RNA was isolated of each IP sample and sequenced in parallel with the input sample.
Figure 2C is a volcano plot of transcript abundance in PHGDH-IP vs. input *(left panel)* and isotype-IP vs. input (*right panel*). Red (*upper*) dots indicate significantly differentially expressed transcripts with p_{adj}<0.05, whereas blue (*lower*) dots represent transcripts without significant enrichment after IP. From the 927 transcripts enriched in the isotype-IP, 637 were also enriched in the PHGDH-IP.
Figure 2D is a scatterplot of RIPseq data showing the enrichment over input for each transcript using PHGDH-IP (x-axis) vs. isotype-IP (y-axis). Pull-down was defined as specific when RNA species were (i) enriched >2 fold in the PHGDH-IP over input, and (ii) enriched 2-times more in the PHGDH-IP compared to the isotype-IP.
Figure 2E shows relative abundance of pro-survival genes in PHGDH (*left bars*) and Isotype (*right bars*) IP samples detected in RIPseq experiment.
Figure 2F is a schematic of RNAseq experiment after CRISPR-Cas9 induced gene-editing of PHGDH (n=5).
Figure 2G is a volcano plot of differential expressed transcripts (RNAseq after gene-editing) in PHGDH expressing vs. PHGDH depleted primary effector T cells 72h post-activation.
Figure 2H is a Venn diagram of hits detected by RNAseq- and RIPseq experiments. The overlap represents transcripts defined as PHGDH targets by RIPseq, which were also functionally responsive upon PHGDH depletion (RNAseq). Transcripts linked to the regulation of gene expression, development and survival are highlighted in the box.
Figure 3A shows PHGDH mRNA expression according to the ImmGen database among splenic (Sp) murine T cell subsets. Normalized expression values for naive (T.8.Nve.Sp), LCMV specific central memory (T8.Tcm.LCMV.d180.Sp) and effector memory CD8⁺ T cells (T8.Tem.LCMV.d180.Sp) are shown. Annotations are replicated from ImmGen.
Figure 3B shows sorting strategy for murine CD8⁺ T_{NV} and T_{EM} cells based on expression of CD62L and CD44 *(left panel).* Bar graph depicts PHGDH levels quantified by intracellular flow cytometry among splenic TNV and TEM cells (n=5 mice, *middle panel*). Western blot of the SSP-enzymes in sorted, non-activated murine T_{NV} and T_{EM} cells (pooled cells of 5 mice, *right panel*).
Figure 3C is a schematic of the adoptive transfer experiment using OVA-specific OT-I T cells (Thy1.1⁺) transduced with an empty vector (EV) or a vector encoding for PHGDH (OE-PHGDH).
Figure 3D shows a Western blot analyzing PHGDH expression in OT-I T cells transduced with EV or OE-PHGDH prior adoptive transfer.
Figure 3E shows frequency of Thy1.1⁺GFP⁺T cells transduced with EV or OE-PHGDH over the course of LmOva infection (n=8-9, *left panel*). Bar graph representing frequencies of Thy1.1⁺GFP⁺T cells at 7 and 14 dpi (*middle and right panel*).
Figure 3F shows frequency of Thy1.1⁺GFP⁺T cells transduced with EV (*lower line*), wild type PHGDH (OE-WT) (*middle line*) or catalytic dead PHGDH (OE-CD) (*upper line*) over the course of LmOVA infection (n=5).
Figure 3G shows frequency of Thy1.1⁺GFP⁺T cells transduced with EV or OE-CD over the course of LmOVA infection followed by reinfection 35 dpi (n=5). Data are represented as mean +SD (B) or ± SEM. P values were determined using an unpaired two-tailed Student's t-test
Figure 4A is a representative histogram of PHGDH expression in T_{NV} electroporated with negative-gRNA (sg-Cont) (*right peaks*) or PHGDH-gRNA (sg-PHGDH) (*left peaks*) and then activated for 96 hours using an anti-CD3/CD28 mAb.
Figure 4B is a dot plot depicting gating strategy used to discriminate viable, late and early apoptotic cells in T_{NV} electroporated with sg-Cont or sg-PHGDH 96h post-activation (*left panel*). Frequency of viable, late and early apoptotic cells in T_{NV} electroporated with sg-Cont (*left bars*) or sg-PHGDH (*right bars*) 96h post-activation (n=3, *right panel).*
Figure 4C is a dot plot depicting gating strategy used to sort CD19 CAR T cells (CAR only) and CD19 CAR_PHGDH (CD) T cells (CAR_PHGDH (CD)) according to GFP and mCherry expression *(left panel).* Relative cell number of CD19 CAR T cells and CD19 CAR_PHGDH (CD) T cells normalized to CD19 CAR T cell number at 2-4 days (n=3-4, *right panel).*
Figure 4D shows a Western blot analyzing PHGDH expression in CD19 CAR T cells and CD19 CAR_PHGDH T cells (n=2).
Figure 4E is a schematic of experimental design *(left panel).* Frequency of CD3⁺CD45⁺ T cells 8 days post transfer (n=4-8, *right panel).* Data are presented as mean ± SEM. P values were determined using an unpaired two-tailed Student's t-test. ^{∗}P<0.05, ^{∗∗}P<0.01.

### Detailed description

The invention is based on the finding that phosphoglycerate dehydrogenase (PHGDH) is differentially expressed in naïve T cells (T_{NV}) compared to effector memory T cells (T_{EM}). Surprisingly, it was identified that PHGDH was expressed in T_{NV} but not T_{EM} cells. More specifically, the inventors have shown that PHGDH expression is downregulated during the effector to memory transition of CD8⁺ T cells. The inventors have identified a non-metabolic role for PHGDH as an RNA binding protein (RBP), exhibiting RNA binding activity, which is implicated in the regulation of cell differentiation and survival. The inventors have shown that enhanced persistence of a cellular therapy can be achieved *in vitro* and *in vivo* by enforcing PHGDH expression in the cells to be transplanted, such as immune cells to be transplanted as part of an adoptive immune cell therapy, in particular T cells, preferably CAR T cells.

The invention thus addresses the above-mentioned needs in the art. For example, the invention addresses needs in the art of cellular therapies by providing an improved cellular therapy, expression constructs, vectors and methods for preparing and using the same. In particular, the invention addresses problems of insufficient cell persistence in the field of regenerative medicine, in particular insufficient cell persistence of cellular therapies, preferably adoptive cell therapies, preferably CAR T cell therapy. The invention provides a cellular therapy that improves the persistence of the cells to be transplanted. The invention provides an improved cellular therapy in applications where it is important to increase the persistence of transplanted cells. In a preferred aspect, the invention provides an improved adoptive immune cell therapy, wherein the immune cell is preferably a lymphocyte, preferably a T cell, preferably a CAR T cell. The invention thus provides an improved cancer therapy or an improved treatment for an infection.

Phosphoglycerate dehydrogenase (PHGDH) is an enzyme that catalyzes the first - and rate-limiting - step of the L-serine biosynthesis pathway (SSP) in animal cells (also referred to herein as L-serine biosynthesis pathway catalytic enzyme activity or PHGDH catalytic activity). PHGDH can comprise or consist of the following amino acid sequence in humans:
Human WT PHGDH (SEQ ID NO: 1) sp|043175|SERA HUMAN D-3-phosphoglycerate dehydrogenase OS=Homo sapiens OX=9606 GN=PHGDH PE=1 SV=4
Human WT PHGDH can be encoded by the following *PHGDH* gene in humans: Human *PHGDH* (WT) - NCBI Reference Sequence: NM 006623.4 (SEQ ID NO: 2)

ATGAAGCTTATGGCTTTTGCAAATCTGCGGAAAGTGCTCATCAGTGACAGCCTGGACCCTTGCTGCC
GGAAGATCTTGCAAGATGGAGGGCTGCAGGTGGTGGAAAAGCAGAACCTTAGCAAAGAGGAGCTG
ATAGCGGAGCTGCAGGACTGTGAAGGCCTTATTGTTCGCTCTGCCACCAAGGTGACCGCTGATGTCA
TCAACGCAGCTGAGAAACTCCAGGTGGTGGGCAGGGCTGGCACAGGTGTGGACAATGTGGATCTG
GAGGCCGCAACAAGGAAGGGCATCTTGGTTATGAACACCCCCAATGGGAACAGCCTCAGTGCCGCA
GAACTCACTTGTGGAATGATCATGTGCCTGGCCAGGCAGATTCCCCAGGCGACGGCTTCGATGAAG
GACGGCAAATGGGAGCGGAAGAAGTTCATGGGAACAGAGCTGAATGGAAAGACCCTGGGAATTCT
TGGCCTGGGCAGGATTGGGAGAGAGGTAGCTACCCGGATGCAGTCCTTTGGGATGAAGACTATAG
GGTATGACCCCATCATTTCCCCAGAGGTCTCGGCCTCCTTTGGTGTTCAGCAGCTGCCCCTGGAGGA
GATCTGGCCTCTCTGTGATTTCATCACTGTGCACACTCCTCTCCTGCCCTCCACGACAGGCTTGCTGA
ATGACAACACCTTTGCCCAGTGCAAGAAGGGGGTGCGTGTGGTGAACTGTGCCCGTGGAGGGATC
GTGGACGAAGGCGCCCTGCTCCGGGCCCTGCAGTCTGGCCAGTGTGCCGGGGCTGCACTGGACGT
GTTTACGGAAGAGCCGCCACGGGACCGGGCCTTGGTGGACCATGAGAATGTCATCAGCTGTCCCCA
CCTGGGTGCCAGCACCAAGGAGGCTCAGAGCCGCTGTGGGGAGGAAATTGCTGTTCAGTTCGTGG
ACATGGTGAAGGGGAAATCTCTCACGGGGGTTGTGAATGCCCAGGCCCTTACCAGTGCCTTCTCTCC
ACACACCAAGCCTTGGATTGGTCTGGCAGAAGCTCTGGGGACACTGATGCGAGCCTGGGCTGGGTC
CCCCAAAGGGACCATCCAGGTGATAACACAGGGAACATCCCTGAAGAATGCTGGGAACTGCCTAAG
CCCCGCAGTCATTGTCGGCCTCCTGAAAGAGGCTTCCAAGCAGGCGGATGTGAACTTGGTGAACGC
TAAGCTGCTGGTGAAAGAGGCTGGCCTCAATGTCACCACCTCCCACAGCCCTGCTGCACCAGGGGA
GCAAGGCTTCGGGGAATGCCTCCTGGCCGTGGCCCTGGCAGGCGCCCCTTACCAGGCTGTGGGCTT

GGTCCAAGGCACTACGCCTGTACTGCAGGGGCTCAATGGAGCTGTCTTCAGGCCAGAAGTGCCTCT
CCGCAGGGACCTGCCCCTGCTCCTATTCCGGACTCAGACCTCTGACCCTGCAATGCTGCCTACCATGA
TTGGCCTCCTGGCAGAGGCAGGCGTGCGGCTGCTGTCCTACCAGACTTCACTGGTGTCAGATGGGG
AGACCTGGCACGTCATGGGCATCTCCTCCTTGCTGCCCAGCCTGGAAGCGTGGAAGCAGCATGTGA
CTGAAGCCTTCCAGTTCCACTTCTAAGGATCC

PHGDH can comprise one or more mutations that reduce L-serine biosynthesis pathway catalytic enzyme activity or completely knock out such activity (so-called "catalytically dead" variants of PHGDH - "PHGDH (CD)"). PHGDH catalytic activity can be measured using techniques known in the art, for example using a commercial PHGDH catalytic activity kit (Abcam, ab273328), or as described herein. PHGDH can comprise one or more mutations in the catalytic domain of PHGDH to reduce or knock out PHGDH catalytic activity. For example, PHGDH can be a PHGDH (CD) comprising a R236E mutation (as described in Mattaini et al. Cancer & Metabolism (2015) 3:5; DOI: 10.1186/s40170-015-0131-7). PHGDH (CD) can comprise or consist of the following amino acid sequence in humans:
PHGDH (CD) R236E aa sequence (SEQ ID NO: 3), R236E mutation is highlighted 
MAFANLRKVLISDSLDPCCRKILQDGGLQVVEKQNLSKEELIAELQDCEGLIVRSATKVTADVINAAEKLQVVGR
AGTGVDNVDLEAATRKGILVMNTPNGNSLSAAELTCGMIMCLARQIPQATASMKDGKWERKKFMGTELNGKTLGI
LGLGRIGREVATRMQSFGMKTIGYDPIISPEVSASFGVQQLPLEEIWPLCDFITVHTPLLPSTTGLLNDNTFAQC
KKGVRVVNCAEGGIVDEGALLRALQSGQCAGAALDVFTEEPPRDRALVDHENVISCPHLGASTKEAQSRCGEEIA
VQFVDMVKGKSLTGVVNAQALTSAFSPHTKPWIGLAEALGTLMRAWAGSPKGTIQVITQGTSLKNAGNCLSPAVI
VGLLKEASKQADVNLVNAKLLVKEAGLNVTTSHSPAAPGEQGFGECLLAVALAGAPYQAVGLVQGTTPVLQGLNG
AVFRPEVPLRRDLPLLLFRTQTSDPAMLPTMIGLLAEAGVRLLSYQTSLVSDGETWHVMGISSLLPSLEAWKQHV
TEAFQFHF
PHGDH (CD) R236E can be encoded by the following sequence: Human *PHGDH* (CD) (SEQ ID NO: 4)

ATGAAGCTTATGGCTTTTGCAAATCTGCGGAAAGTGCTCATCAGTGACAGCCTGGACCCTTGCTGCC
GGAAGATCTTGCAAGATGGAGGGCTGCAGGTGGTGGAAAAGCAGAACCTTAGCAAAGAGGAGCTG
ATAGCGGAGCTGCAGGACTGTGAAGGCCTTATTGTTCGCTCTGCCACCAAGGTGACCGCTGATGTCA
TCAACGCAGCTGAGAAACTCCAGGTGGTGGGCAGGGCTGGCACAGGTGTGGACAATGTGGATCTG
GAGGCCGCAACAAGGAAGGGCATCTTGGTTATGAACACCCCCAATGGGAACAGCCTCAGTGCCGCA

GAACTCACTTGTGGAATGATCATGTGCCTGGCCAGGCAGATTCCCCAGGCGACGGCTTCGATGAAG
GACGGCAAATGGGAGCGGAAGAAGTTCATGGGAACAGAGCTGAATGGAAAGACCCTGGGAATTCT
TGGCCTGGGCAGGATTGGGAGAGAGGTAGCTACCCGGATGCAGTCCTTTGGGATGAAGACTATAG
GGTATGACCCCATCATTTCCCCAGAGGTCTCGGCCTCCTTTGGTGTTCAGCAGCTGCCCCTGGAGGA
GATCTGGCCTCTCTGTGATTTCATCACTGTGCACACTCCTCTCCTGCCCTCCACGACAGGCTTGCTGA
ATGACAACACCTTTGCCCAGTGCAAGAAGGGGGTGCGTGTGGTGAACTGTGCCGAGGGAGGGATC
GTGGACGAAGGCGCCCTGCTCCGGGCCCTGCAGTCTGGCCAGTGTGCCGGGGCTGCACTGGACGT
GTTTACGGAAGAGCCGCCACGGGACCGGGCCTTGGTGGACCATGAGAATGTCATCAGCTGTCCCCA
CCTGGGTGCCAGCACCAAGGAGGCTCAGAGCCGCTGTGGGGAGGAAATTGCTGTTCAGTTCGTGG
ACATGGTGAAGGGGAAATCTCTCACGGGGGTTGTGAATGCCCAGGCCCTTACCAGTGCCTTCTCTCC
ACACACCAAGCCTTGGATTGGTCTGGCAGAAGCTCTGGGGACACTGATGCGAGCCTGGGCTGGGTC
CCCCAAAGGGACCATCCAGGTGATAACACAGGGAACATCCCTGAAGAATGCTGGGAACTGCCTAAG
CCCCGCAGTCATTGTCGGCCTCCTGAAAGAGGCTTCCAAGCAGGCGGATGTGAACTTGGTGAACGC
TAAGCTGCTGGTGAAAGAGGCTGGCCTCAATGTCACCACCTCCCACAGCCCTGCTGCACCAGGGGA
GCAAGGCTTCGGGGAATGCCTCCTGGCCGTGGCCCTGGCAGGCGCCCCTTACCAGGCTGTGGGCTT
GGTCCAAGGCACTACGCCTGTACTGCAGGGGCTCAATGGAGCTGTCTTCAGGCCAGAAGTGCCTCT
CCGCAGGGACCTGCCCCTGCTCCTATTCCGGACTCAGACCTCTGACCCTGCAATGCTGCCTACCATGA
TTGGCCTCCTGGCAGAGGCAGGCGTGCGGCTGCTGTCCTACCAGACTTCACTGGTGTCAGATGGGG
AGACCTGGCACGTCATGGGCATCTCCTCCTTGCTGCCCAGCCTGGAAGCGTGGAAGCAGCATGTGA
CTGAAGCCTTCCAGTTCCACTTCTAAGGATCC

Metabolic enzymes can also engage in non-metabolic or "moonlighting" roles, which creates potential for functional incompatibilities at the cellular level. The inventors have identified a non-metabolic or moonlighting role for PHGDH. In particular, the invention is based on the finding that PHGDH is silenced during effector T cell contraction. The inventors have shown that PHGDH has a moonlighting role as an RNA binding protein, exhibiting RNA binding activity, and this property contributes to regulating persistence in T cells. The inventors have shown how catalytically dead variants of PHGDH, for example PHGDH (CD) R236E, can retain RNA binding activity. Investigations with such variants helped elucidate PHGDH's non-metabolic RNA binding role.

In one aspect, the invention provides a cell genetically engineered to enforce expression of PHGDH.

The term "enforced expression" and similar terms refers to both the transcription of a gene or transgene into a messenger RNA (mRNA) by a cell and translation of that mRNA into a polypeptide (i.e., the gene or transgene product). Expression of a gene or transgene can be measured by techniques known in the art at the mRNA level (for example, by sequence-specific quantification of the level of mRNA in a cell) or at the polypeptide level (for example, by measuring the level of polypeptide gene product using a Western Blot, densitometry, Enzyme-Linked Immunoabsorbent Assay (ELISA), Immunofluorescence Microscopy, or other means of quantifying a specific polypeptide). By "enforced expression" is meant expression of a gene, transgene or polypeptide that is not expressed under normal conditions or expression of a gene or polypeptide at an amount greater than that expressed under normal conditions for a given cell, for example any of the cells described herein. Enforced expression can be measured by comparing a test cell (for example, a cell genetically engineered as described herein) with a reference or control cell (for example, a cell that has not been genetically engineered as described herein or has been genetically engineered but includes an empty vector or does not otherwise include a transgene as descried herein) under the same or similar conditions. Suitably, a control cell is derived from the same population of cells as the test cell, wherein the population of cells is for use in a cellular therapy, wherein the test cells are genetically engineered as described herein. Enforced expression of PHGDH can be a measure of the expression of PHGDH mRNA and/or polypeptide greater than 0 in a cell or cells that do not express PHGDH, for example T_{EM} as described herein. Enforced expression of PHGDH can be a measure of increased expression of PHGDH mRNA and/or polypeptide in a test cell or cells compared to a control cell or cells, suitably greater than a 1-fold increase, such as at least a 1.1-fold increase or more, a 1.2-, 1.3-, 1.4-, 1.5-, 1.6-, 1.7-, 1.8-, 1.9-, 2-, 3-, 4-, 5-, or at least a 10-fold increase in expression of PHGDH mRNA and/or polypeptide in the test cell or cells.

As used herein, the singular forms "a", "an" and "the" are intended to include the plural forms as well, unless the context clearly indicates otherwise.

Expression of PHGDH can be enforced by genetically engineering a cell to incorporate an exogenous polynucleotide sequence encoding PHGDH (for example, using a vector comprising an exogenous polynucleotide sequence encoding PHGDH) or by altering endogenous expression of PHGDH (for example, by preventing or reducing the silencing of PHGDH expression or by increasing the expression of endogenous PHGDH, for example by increasing the activity of one or more endogenous promoter and/or enhancer regions associated with the *PHGDH* gene).

The data provided herein show that PHGDH can exhibit a moonlighting role as an RNA binding protein (RBP), having RNA binding activity. PHGDH has been shown to exhibit RNA binding activity in T cells, activity which is implicated in the regulation of cell differentiation and survival. PHGDH expression can therefore promote persistence of cells and/or prevent cells from dying away, for example reducing the apoptosis of effector T cells during a contraction phase.

RNA binding activity can be determined by methods known in the art and as disclosed herein, for example using one or more RNA electrophoretic mobility shift assay (EMSA), RNA pull-down assay including RNA immunoprecipitation sequencing (RIPseq) and RNA sequencing (RNAseq), oligonucleotide-targeted RNase H protection assays and Fluorescent in situ hybridization co-localization assays. In a preferred aspect, PHGDH as described herein has RNA binding activity, preferably wherein PHGDH binds one or more mRNAs encoding transcripts regulating pro-apoptotic and/or pro-survival processes, and/or one or more mRNAs encoding epigenetic modifiers and/or components of WNT signaling within a cell, preferably a cell as described herein, preferably a T cell. In a preferred aspect, PHGDH binds one or more mRNAs encoding transcripts selected from BBC3, MCL1, TP53BP2, NFKBIA, IRF1, BCL2, KDM3A, DOT1L, KAT2A, KDM6B, DVL1 and Jun; preferably one or more mRNAs encoding transcripts regulating pro-apoptotic and/or pro-survival processes, preferably selected from BBC3, MCL1, TP53BP2, NFKBIA, IRF1, and BCL2. The mRNAs can be detected by methods known in the art and as described herein, preferably a combination of RIPseq and RNAseq.

In one aspect, PHGDH as described herein has RNA binding activity, wherein PHGDH binds to RNAs that are (i) enriched >2 fold in the PHGDH-IP over input (total cell lysate), and (ii) enriched 2-times more in the PHGDH-IP when compared to control (isotype-IP) in the RIPseq assay described herein. Suitably, PHGDH binds to about 200 or more, for example about 300, 400, 500, 600, 700, 800, 900, 1000, 1500, or about 2000 or more RNAs, preferably about 1500 or more protein coding RNAs in the RIPseq assay described herein, preferably in T cells.

In one aspect, PHGDH can have RNA binding activity but no or reduced PHGDH catalytic activity. In a preferred aspect, PHGDH can comprise one or more mutations in the catalytic domain of PHGDH to reduce or knock out PHGDH catalytic activity but retain RNA binding activity as described herein, preferably one or more mutations that render the PHGDH catalytically dead but retaining RNA binding activity, preferably PHGDH (CD) comprising the amino acid sequence of SEQ ID NO: 3, wherein the PHGDH has RNA binding activity.

PHGDH as described herein can comprise or consist of an amino acid sequence of SEQ ID NO: 1 or 3 or a sequence having at least 80%, at least 85%, at least 90%, at least 95%, at least 96%, at least 97%, at least 98%, or at least 99% identity to SEQ ID NO: 1 or 3, wherein the polypeptide has RNA binding activity as described herein, or a functional fragment or variant thereof. Preferably, PHGDH as described herein comprises or consists of SEQ ID NO: 3 or a sequence having at least 80%, at least 85%, at least 90%, at least 95%, at least 96%, at least 97%, at least 98%, or at least 99% identity to SEQ ID NO: 3, wherein the polypeptide has RNA binding activity, or a functional fragment or variant thereof.

In one aspect, the invention provides a cell genetically engineered to enforce expression of PHGDH, wherein the cell is for a cellular therapy as described herein. The invention provides a cell genetically engineered to enforce expression of PHGDH, wherein the cell is useful or can be used in a cellular therapy or method as described herein.

Cellular therapy is a therapy in which viable cells are provided or administered to a subject. Cells can be transplanted, for example injected, grafted or infused, into a subject in order to treat a condition or disease. It is desirable to enhance the persistence of cells to be transplanted into a subject as part of a cellular therapy in order to treat the condition or disease, in particular in adoptive cellular therapies such as CAR T cell therapy.

A cell as described herein can be any cell useful in regenerative medicine, for example a cellular therapy as described herein. In a preferred aspect, a cell as described herein can be any cell useful in regenerative medicine that would benefit from an increase in the persistence of the cells, preferably a cellular therapy, preferably an adoptive cellular therapy that would benefit from an increase in the persistence of the transplanted cells. In one aspect, the cell can be any cell useful in regenerative medicine, wherein PHGDH expression is downregulated or silenced with differentiation, preferably a cellular therapy, preferably an adoptive cellular therapy. Downregulation or silencing of PHGDH expression with differentiation can be determined by methods known in the art and as described herein, such as Western blot, real time reverse transcriptase PCR, epigenetic analysis. In a preferred aspect, the cell can be an immune cell, a neuron, or a breast-, prostate- or colonic-epithelial cell. In one aspect, the cell can be any cell useful in regenerative medicine that would benefit from regulation of apoptosis and/or pro-survival processes, preferably a cellular therapy, preferably an adoptive cellular therapy.

Adoptive cellular therapy, also known as cellular immunotherapy, is a form of cellular therapy that uses the cells of the mammalian immune system, preferably the human immune system, to treat a condition or disease, in particular cancer. In a preferred aspect, any of the cells described herein, in particular a cell genetically engineered to enforce expression of PHGDH as described herein, can be any cell used in an adoptive cellular therapy, including Tumor-Infiltrating Lymphocyte (TIL) therapy, Engineered T Cell Receptor (TCR) therapy, Chimeric Antigen Receptor (CAR) T cell therapy or Natural Killer (NK) cell therapy, preferably CAR T cell therapy.

The cells of the immune system can be categorized as lymphocytes (including T-cells, B-cells and NK cells), neutrophils, and monocytes/macrophages. A cell of the invention can be any immune cell, preferably a lymphocyte. The term "lymphocyte" as used herein refers to any of the mononuclear non-phagocytic leukocytes found in the blood, lymph, and lymphoid tissues which are derived from lymphoid stem cells. Lymphocytes include T cells, natural killer (NK) cells, and B cells, CIK (cytokine induced killer) cells and combinations thereof.

A T cell is a type of lymphocyte (a T lymphocyte) that develops in the thymus gland and plays a central role in the immune response. T cells can be distinguished from other lymphocytes by the presence of a T cell receptor on the cell surface. These immune cells originate as precursor cells, derived from bone marrow, and develop into several distinct types of T cells once they have migrated into the thymus gland. T cell differentiation continues after they have left the thymus. A cell of the invention can be any T cell, for example a CD3⁺ cell, a helper CD4⁺ T cell, a cytotoxic CD8⁺ T cell, a memory T cell, a regulatory CD4⁺ T cell, a natural killer T cell or a gamma delta T cell or combination thereof, preferably a cytotoxic CD8⁺ T cell.

Natural killer cells, or NK cells, are a type of cytotoxic lymphocyte critical to the innate immune system. The role NK cells play is analogous to that of cytotoxic T cells in the vertebrate adaptive immune response. NK cells provide rapid responses to virus-infected cells and respond to tumor formation.

Tumor infiltrating lymphocytes (TILs) are white blood cells that have left the bloodstream and migrated towards a tumor. They include T cells and B cells and are part of the larger category of 'tumor-infiltrating immune cells' which consist of both mononuclear and polymorphonuclear immune cells, (e.g., T cells, B cells, natural killer cells, macrophages, neutrophils, dendritic cells, mast cells, eosinophils, basophils, etc.) in variable proportions. Their abundance varies with tumor type and stage and in some cases relates to disease prognosis. TILs may be used in cell therapy, wherein TILs are isolated from a patient's tumor and expanded *ex vivo.* The expanded TILs may then be assayed for specific tumor recognition and the tumor specific TILs may then be re-infused into the patient, optionally after an additional expansion step.

In a preferred aspect, a cell as described herein, in particular a cell genetically engineered to enforce expression of PHGDH as described herein, is a T cell, NK cell, B cell or a combination thereof; preferably a T cell or an NK cell, preferably a T cell, preferably a CD3⁺ cell, preferably a CD8⁺ T cell and/or a CD4⁺ T cell. In various aspects, the cell can comprise a CAR or a modified TCR. In various aspects, the cell can be an antigen-specific cell, preferably a tumour antigen- or viral antigen-specific cell, preferably an antigen-specific T cell.

In one aspect, the invention concerns a plurality of cells as described herein, in particular a plurality of cells genetically engineered to enforce expression of PHGDH as described herein.

In a preferred aspect, the plurality of cells can comprise a plurality of T cells, NK cells, B cells or a combination thereof; preferably T cells, NK cells or a combination thereof; preferably a plurality of T cells, preferably CD3⁺ cells, preferably CD8⁺T cells and/or a CD4⁺T cells, optionally comprising a CAR.

A cell or cells of the invention can be allogeneic (wherein cells are derived from a subject different to the subject receiving the transplanted cells), autologous (wherein cells are derived from the subject's own tissues), or xenogeneic (wherein cells are derived from another species). In a preferred aspect, the cell is autologous.

In one aspect, the cell or cells of the invention can be genetically engineered to enforce expression of PHGDH by incorporating exogenous polynucleotide encoding PHGDH (a "*PHGDH* transgene") into the cells. In another aspect, the cells of the invention can be genetically engineered to enforce expression of PHGDH by enhancing endogenous expression of PHGDH. For example, the expression of endogenous PHGDH can be increased by altering the activity of one or more regulatory element, for example increasing the activity of one or more endogenous promoter and/or enhancer or inhibiting suppressor regions associated with the PHGDH gene by methods known in the art. For example, the promoter of the endogenous gene encoding PHGDH may be modified such that the gene encoding PHGDH is constitutively expressed in the cell. Similarly, silencing of endogenous PHGDH expression can be prevented or reduced by inhibiting epigenetic silencing of PHGDH. Methods for genetic engineering are well known in the art and include CRISPR/Cas9, or the use of engineered nucleases such as meganucleases, zinc finger nucleases or TALENs.

Exogenous polynucleotide, for example a polynucleotide sequence encoding PHGDH as described herein, can be incorporated into one or more cells, for example one or more cells of the invention as described herein, and expressed in said cells by techniques known in the art and as disclosed herein. Methods for incorporating polynucleotides, such as genes and transgenes, into cells and expressing the genes and transgenes are known in the art and include physical, chemical and biological methods. Physical methods for introducing a polynucleotide into a host cell include calcium phosphate precipitation, lipofection, particle bombardment, microinjection, electroporation. Chemical methods for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. Biological methods for introducing a polynucleotide of interest into a host cell include the use of viral vectors.

The practice of the present invention employs, unless otherwise indicated, conventional techniques of cell biology, molecular biology (including recombinant techniques), microbiology, biochemistry and immunology, which are within the scope of those of skill in the art. Such techniques are explained fully in the literature, such as, "Molecular Cloning: A Laboratory Manual", second edition (Sambrook et al., 1989); "Oligonucleotide Synthesis" (M. J. Gait, ed., 1984); "Animal Cell Culture" (R. I. Freshney, ed., 1987); "Methods in Enzymology" (Academic Press, Inc.); "Handbook of Experimental Immunology" (D. M. Weir & C. C. Blackwell, eds.); "Gene Transfer Vectors for Mammalian Cells" (J. M. Miller & M. P. Calos, eds., 1987); "Current Protocols in Molecular Biology" (F. M. Ausubel et al., eds., 1987); "PCR: The Polymerase Chain Reaction", (Mullis et al., eds., 1994); and "Current Protocols in Immunology" (J. E. Coligan et al., eds., 1991), each of which is expressly incorporated by reference herein.

The term "transgene" as used herein refers to a polynucleotide sequence to be inserted into a cell that encodes a polypeptide or a portion of a polypeptide to be expressed in the cell. A PHGDH transgene can include a nucleic acid sequence that is not naturally found in the cell (*i.e*., a heterologous nucleic acid sequence encoding PHGDH); a nucleic acid sequence that is a variant or mutant form of a nucleic acid sequence naturally found in the cell; or a nucleic acid sequence naturally occurring in the cell.

The term "encoding" refers to the inherent property of specific sequences of nucleotides in a polynucleotide, such as a coding sequence, a gene, a cDNA, or an RNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a coding sequence or a gene encodes a protein if transcription of the coding sequence or the gene into mRNA and translation of mRNA corresponding to that coding sequence or gene produces the protein in a cell or other biological system.

A "vector" as used herein refers to a macromolecule or association of macromolecules that comprises or associates with a polynucleotide and which can mediate delivery of the polynucleotide to a cell. Illustrative vectors include, for example, plasmids, viral vectors, liposomes, and other gene delivery vehicles. For example, viral vector approaches such as retroviral and lentiviral (LV) vectors utilize numerous vectors and are well characterized in the art. One or more plasmid vectors can be used to transfect cells, preferably *in vitro* (for example in HEK-293 cells), to generate viral particles (viral vectors), for example viral vectors comprising an expression cassette as described herein (an expression vector).

A vector comprising a polynucleotide encoding a gene product of interest and effecting the expression of a gene product in an intended target cell can be referred to as an "expression vector". An expression vector can comprise control elements (*e*.*g*., promoters, enhancers, UTRs, miRNA targeting sequences, likers, tags, etc.) operatively linked to the polynucleotide or region encoding the gene product of interest to facilitate expression of the gene product in the target cell. The combination of control elements and polynucleotide or region encoding the gene product of interest to which they are operably linked for expression is referred to herein as an "expression cassette". Many such control elements are known and available in the art or can be readily constructed from components that are available in the art.

Any of the cells, expression cassettes and vectors as described herein can comprise a polynucleotide sequence encoding PHGDH. Suitably, PHGDH is PHGDH WT or PHGDH (CD) having RNA binding activity, preferably PHGDH is PHGDH (CD) having RNA binding activity. In various aspects, the polynucleotide sequence comprises SEQ ID NO: 2 or 4 or a sequence having at least 80%, at least 85%, at least 90%, at least 95%, at least 96%, at least 97%, at least 98%, or at least 99% identity to SEQ ID NO: 2 or 4, wherein the sequence encodes a polypeptide having RNA binding activity, or a functional fragment or variant thereof. In a preferred aspect, the polynucleotide sequence encoding PHGDH comprises SEQ ID NO: 4 or a sequence having at least 80%, at least 85%, at least 90%, at least 95%, at least 96%, at least 97%, at least 98%, or at least 99% identity to SEQ ID NO: 4, wherein the sequence encodes a polypeptide having RNA binding activity, or a functional fragment or variant thereof.

By "variant" or "mutant" as used herein is meant a polynucleotide or polypeptide having less than 100% sequence identity with a reference polynucleotide or polypeptide, for example a "wild-type" polynucleotide or polypeptide or polynucleotide or polypeptide naturally found in the cell. A variant or mutant polynucleotide can comprise at least one nucleotide difference (*e*.*g*., nucleotide substitution, nucleotide insertion, or nucleotide deletion) relative to a reference polynucleotide sequence; a variant or mutant polypeptide can comprise at least one amino acid difference (*e*.*g*., amino acid substitution, amino acid insertion, amino acid deletion) relative to a reference polypeptide sequence. Preferably, a variant or mutant polynucleotide encoding PHGDH or a variant or mutant PHGDH polypeptide retains biological activity of wild-type PHGDH, including L-serine biosynthesis pathway catalytic enzyme activity and/or RNA binding activity; preferably RNA binding activity.

In some aspects, the invention provides an expression cassette comprising a polynucleotide sequence encoding phosphoglycerate dehydrogenase PHGDH as described herein. The expression cassette can be adapted for genetically modifying a cell, for example any of the cells useful in a cellular therapy as described herein, preferably an immune cell, preferably a T cell. The expression cassette can be adapted for enforcing expression of PHGDH in a cell, for example any of the cells useful in a cellular therapy as described herein, preferably an immune cell, preferably a T cell. The expression cassette can be adapted for genetically modifying a cellular therapy as described herein, preferably an adoptive cellular therapy, preferably a T cell therapy.

The expression cassette of the invention can comprise a polynucleotide sequence encoding PHGDH as described herein and a control element that controls expression of PHGDH in a cell, for example any of the cells described herein, preferably cells useful in a cellular therapy as described herein, preferably an immune cell, preferably a T cell. The control element can be selected from one or more of a promoter, an enhancer, miRNA targeting sequence, linker, and a tag, preferably wherein the control element is operatively linked to the polynucleotide or region encoding the gene product of interest.

The expression cassette of the invention can increase expression of PHGDH in the cells of the disclosure, preferably by expressing a *PHGDH* transgene in the cells. As used herein "expression of a transgene" or "expressing a transgene" refers to both the transcription of the transgene into a messenger RNA (mRNA) by the host cell and translation of that mRNA into a polypeptide (*i*.*e*., the transgene product). Thus, expression of PHGDH can be measured at the mRNA level (*i*.*e*., by sequence-specific quantification of the level of mRNA in a cell) or at the polypeptide level (*i*.*e*., by measuring the level of polypeptide gene product using a Western Blot, Enzyme-Linked Immunoabsorbent Assay (ELISA), Immunofluorescence Microscopy, or other means of quantifying a specific polypeptide).

The PHGDH transgene can be modified, or "codon optimized", to enhance expression by replacing infrequently represented codons with more frequently represented codons. The coding sequence is the portion of the mRNA sequence that encodes the amino acids for translation. During translation, each of 61 trinucleotide codons are translated to one of 20 amino acids, leading to a degeneracy, or redundancy, in the genetic code. However, different cell types, and different animal species, utilize tRNAs (each bearing an anticodon) coding for the same amino acids at different frequencies. When a gene sequence contains codons that are infrequently represented by the corresponding tRNA, the ribosome translation machinery may slow, impeding efficient translation. Expression can be improved via "codon optimization" for a particular species, where the coding sequence is altered to encode the same protein sequence, but utilizing codons that are highly represented, and/or utilized by highly expressed human proteins (Cid-Arregui et al., 2003; J. Virol. 77: 4928).

A "promoter" as used herein encompasses a nucleotide sequence that directs the binding of RNA polymerase and thereby promotes RNA synthesis (*i*.*e*., initiates transcription). Promoters and corresponding protein or polypeptide expression may be ubiquitous (meaning strongly active in a wide range of cells, tissues and species) or cell-type specific, tissue-specific, or species specific. Promoters may be "constitutive," meaning continually active, or "inducible," meaning the promoter can be activated or deactivated by the presence or absence of biotic or abiotic factors.

An "enhancer" as used herein encompasses a cis-acting element that stimulates or inhibits transcription of adjacent genes. An enhancer that inhibits transcription also is termed a "silencer". Enhancers are nucleic acid elements known in the art to enhance transcription and can be located anywhere in association with the gene they regulate (*e*.*g*., upstream, downstream, within an intron). Enhancers can function in either orientation, over distances of up to several kilobase pairs (kb) from the coding sequence and from a position downstream of a transcribed region. Enhancer sequences influence promoter-dependent gene expression.

Promoter and enhancer elements can be tissue specific or stage-specific. For example, a tissue-specific promoter or enhancer preferentially drives expression (or a higher level of expression) in one or more particular cell types. A stage-specific promoter or enhancer preferentially drives expression (or higher level of expression) during one or more specific stages of the cell cycle or development.

Any suitable promoter, such as a promoter region or promoter sequence therein, can be used in the subject expression cassette, so long as the promoter promotes expression of PHGDH in the cells described herein, preferably eukaryotic cells, preferably mammalian cells, preferably human cells, preferably immune cells, preferably T cells. The promoter can be a constitutive or inducible promoter, preferably an inducible promoter. In a preferred aspect, the promoter can be selected from a CMV, EF1A, EFS, CAG, CBh, SFFV, MSCV, SV40, mPGK, hPGK, UBC or TRE promoter.

Any suitable enhancer can be used in the subject expression cassette, so long as it enhances expression of PHGDH in the cells described herein, in particular when used in combination with any of the promoters described herein.

The polynucleotide sequence encoding PHGDH may be heterologous to the promoter sequence to which it is operably linked (*i*.*e*. not naturally operably associated with it) or endogenous to the promoter sequence to which it is operably linked (*i*.*e*., naturally operably associated with that promoter).

In a preferred aspect, the expression cassette as described herein can comprise a sequence encoding a chimeric antigen receptor (CAR). The invention provides an expression cassette comprising a polynucleotide sequence encoding phosphoglycerate dehydrogenase (PHGDH) and a polynucleotide sequence encoding CAR as described herein. The expression cassette is suitably a polycistronic expression cassette for expressing a plurality of proteins in a single vector. The polynucleotide sequence encoding PHGDH and the polynucleotide sequence encoding the CAR can be separated or flanked by one or more linkers, preferably selected from one or more internal ribosome entry site (IRES) or one or more polynucleotides encoding a "self-cleaving" peptide, such as a self-cleaving 2A peptide selected from P2A, T2A, E2A and F2A, or a combination thereof using techniques known in the art. Coding sequences separated by an IRES sequence are each independently translated. 2A peptides are 18-22 amino-acid long viral oligopeptides that mediate "cleavage" of polypeptides during translation in eukaryotic cells.

The expression cassette of the invention can comprise the following general structure: 5' - promoter - CAR - linker - PHGDH - 3'; 5' - promoter - PHGDH - linker - CAR - 3'; wherein the promoter can be a sequence encoding any of the promoters described herein, the CAR can be a sequence encoding any of the CARs described herein, the PHGDH can be any of the polynucleotide sequences encoding PHGDH as described herein, and the linker can be any of the linker sequences or polynucleotides encoding a linker sequence as described herein. The expression cassette can comprise further polynucleotide sequences at the 5' end, the 3' end and in the regions between one or more of the promoter, PHGDH, linker and CAR sequences. For example, the expression cassette as described herein can comprise one or more tags, preferably at the 3' end, preferably selected from mCherry, GFP, any fluoresecent tag, CD34, FLAG, and C-Myc. The expression cassette can comprise one or more likers as described herein, for example separating the 3' CAR or PHGDH sequence from the tag.

In an alternative aspect, the disclosure relates to an expression cassette as disclosed herein comprising a polynucleotide sequence encoding PHGDH as described herein and a separate expression cassette comprising a sequence encoding a chimeric antigen receptor (CAR) as described herein, wherein the expression cassettes are adapted for genetically modifying a cell as described herein, preferably an immune cell, preferably a T cell. The expression cassettes can be adapted for enforcing expression of PHGDH and expressing a CAR in a cell as described herein, preferably an immune cell, preferably a T cell. The expression cassettes can be adapted for genetically modifying a cellular therapy as described herein, preferably an adoptive cellular therapy, preferably a T cell therapy.

CARs are engineered synthetic receptors that can be expressed in immune cells and that function to redirect the immune cells, preferably lymphocytes, most commonly T cells, to recognize and eliminate cells expressing a specific target antigen. The binding of a CAR to a target antigen expressed on the cell surface is independent of the MHC receptor, resulting in vigorous T cell activation and powerful anti-tumor responses. CARs are modular synthetic receptors, the structure of which has been well characterized in the art. CARs consist of three main components: (1) an extracellular target antigen binding domain (ABD), (2) a transmembrane domain, and (3) one or more intracellular signaling domains. CARs can also comprise a hinge region that extends the ABD from the transmembrane domain.

The antigen binding domain (ABD) (or ectodomain) is the portion of the CAR that confers target antigen specificity. Typically, the antigen-binding domains are derived from the variable heavy (VH) and light (VL) chains of monoclonal antibodies, connected via a flexible linker to form a single chain variable fragment (scFv).

The transmembrane domain connects the ABD and intracellular signaling domain and functions to anchor the CAR to the cell membrane. The transmembrane domain is normally derived from a transmembrane receptor protein. Most transmembrane domains are derived from natural proteins including CD3ζ, CD4, CD8α, or CD28, but the transmembrane domain may also be derived from a synthetic source.

The intracellular signaling domain (or cytoplasmic signaling domain) is responsible for activation of at least one of the normal effector functions of the immune cell in which the CAR is expressed. The "effector function" refers to a specialized function of a cell. Effector function of a T cell, for example, may be cytolytic activity or helper activity including the secretion of cytokines. CARs can comprise one or more intracellular signaling domain (For example, second generation CARs can comprise two intracellular signaling domains; and third generation CARs can comprise three intracellular signaling domains), wherein cell activation is mediated by different classes of intracellular signaling domain: at least one intracellular signaling domain that initiates antigen-dependent primary activation through the TCR (a primary intracellular signaling domain) and one or more intracellular signaling domains that acts in an antigen-independent manner to provide a secondary or co-stimulatory signal (co-stimulatory intracellular signaling domain).

Primary intracellular signaling domains regulate primary activation of the TCR complex either in a stimulatory way, or in an inhibitory way. Primary intracellular signaling domain that act in a stimulatory manner may contain signaling motifs which are known as immunoreceptor tyrosine-based activation motifs (ITAMs).

A co-stimulatory intracellular signaling domain refers to a portion of the CAR comprising the intracellular domain of a costimulatory molecule. A co-stimulatory molecule is a cell surface molecule other than an antigen receptor or their ligands that is required for an efficient response of lymphocytes to an antigen.

The expression cassette as described herein can comprise a sequence encoding a CAR comprising one or more ABD as described herein, a transmembrane domain as described herein, and one or more intracellular signaling domain as described herein, preferably two or more intracellular signaling domains.

The one or more ABD can be any domain known in the art that is capable of specifically binding a specific antigen. In a preferred aspect, the ABD is an antibody or an antigen-binding fragment of an antibody that specifically binds to an antigen, preferably an ScFV. In a preferred aspect, the antigen can be a cancer associated antigen, preferably one or more of BCMA, CD19, CD22, CD30, CD33, CD56, CD123, CEA, EBV-related antigens, EGFR, GD2, GPC3, HER2, HPV-related antigens, MAGE antigens, Mesothelin, MUC-1, NY-ESO-1, PSCA, PSMA, ROR1, WT1, and Claudin 18.2; preferably one or more of CD19, BCMA, CD30, CD33, CD123 or FLT3. In one aspect, the CAR can comprise more than one ABD as described herein, for example two or more ABDs. In one aspect, the CAR can be a bi-specific CAR comprising two or more ABDs, wherein one ABD specifically binds to an antigen as described herein and one ABD specifically binds to a different antigen as described herein; preferably a bispecific CAR comprising a CD19 ABD and a CD22 ABD; a bispecific CAR comprising a CD19 ABD and a CD20 ABD; a bispecific CAR comprising a CD19 ABD and a BCMA ABD; a bispecific CAR comprising a HER2 ABD and a IL13Ra2 ABD; or a bispecific CAR comprising a HER2 ABD and a MUC1 ABD. Approved CAR T cell therapies include CARs comprising an ABD that specifically binds to CD19 or BCMA. In a preferred aspect, the antigen is CD19 or BCMA. The expression cassette as described herein can comprise a sequence encoding a CD19 ScFV, preferably ScFV FM63 (ref.) or a sequence encoding a BCMA ScFV. In another aspect, the antigen can be a viral antigen. The viral antigen may be any component of a virus particle that is accessible to the CAR, for example an antigen that forms part of the surface and/or the protein coat of the virus. Preferably, the viral antigen that is recognized by the CAR is an antigen derived from human immunodeficiency virus (HIV), adenoviruses, polyomaviruses, influenza virus, Hepatitis B, or human herpesvirus, in particular human herpesvirus wherein the human herpesvirus is cytomegalovirus (CMV), Epstein-Barr virus (EBV), herpes simplex vims (HSV), Varizella-Zoster virus (VZV) or human herpesvirus 8 (HHV8), preferably the viral antigen is an EBV antigen.

The transmembrane (TM) domain may be derived from a natural or from a synthetic source. Where the source is natural, the domain may be derived from any membrane-bound or transmembrane protein. The transmembrane region can comprise at least the transmembrane region(s), or a portion thereof, of the alpha, beta or zeta chain of the T-cell receptor, CD28, CD8, CD3 epsilon, CD45, CD4, CD5, CD9, CD16, CD22, CD33, CD37, CD64, CD80, CD86, CD134, CD137, CD154. Alternatively, the transmembrane domain may be synthetic, in which case it will comprise predominantly hydrophobic residues such as leucine and valine. Preferably, a triplet of phenylalanine, tryptophan and valine will be found at each end of a synthetic transmembrane domain.

In a preferred aspect, the transmembrane region is derived from CD8, optionally wherein the hinge region where present is derived from CD8.

The primary intracellular signaling domain can be selected from an intracellular signaling domain derived from CD3 zeta, FcR gamma, FcR beta, CD3 gamma, CD3 delta, CD3 epsilon, CDS, CD22, CD79a, CD79b, and CD66d. In a preferred aspect, the intracellular signaling domain of the CAR described herein comprises a intracellular signaling domain derived from CD3 zeta.

The co-stimulatory intracellular signaling domain can be selected from one or more of CD27, CD28, 4-1BB (CD 137), OX40, CD30, CD40, PD-1, ICOS, lymphocyte function-associated antigen-1 (LFA-1), CD2, CD7, LIGHT, NKG2C, MYD88, B7-H3, and a ligand that specifically binds with CD83. In a preferred aspect the co-stimulatory intracellular signaling domain is CD28 or 4-1BB or a combination thereof.

The CAR as described herein can comprise a short oligo- or polypeptide linker, preferably between 2 and 10 amino acids in length that forms the linkage between the transmembrane domain and the intracellular signaling domain(s) of the CAR, preferably a glycine-serine doublet linker.

In a preferred aspect, an expression cassette according to the invention comprises a polynucleotide sequence encoding a promoter, preferably an EF-1 alpha promoter; a polynucleotide sequence encoding a CAR, preferably wherein the CAR comprises a CD19 ScFV, a CD8 hinge and CD8 TM region, a 4-1BB co-stimulatory region, a CD3 zeta primary intracellular region; a polynucleotide sequence encoding a P2A self-cleaving linker; a polynucleotide sequence encoding PHGDH, preferably comprising SEQ ID NO: 4 or a sequence having 95% identity to SEQ ID NO: 4 and encoding PHGDH having RNA binding activity.

In some aspects, the invention provides a vector comprising any of the polynucleotide sequences described herein or any of the expression cassettes described herein. A vector comprising an expression cassette can be referred to as an expression vector.

Any vector system known in the art and as described herein can be utilized for introducing a polynucleotide sequences as described herein into a cell, preferably a mammalian cell, preferably for expression of the polynucleotide sequence encoding PHGDH as described herein in the cell, preferably an immune cell, preferably a T cell. For example, the vector may comprise single or double stranded nucleic acid, *e*.*g*. single stranded or double stranded DNA. For example, the vector may be DNA, *e.g*., a naked DNA, *e.g*., a plasmid, a minicircle, etc. The vector may comprise single-stranded or double-stranded RNA, including modified forms of RNA. In another example, the vector may be an RNA, *e*.*g*., an mRNA or modified mRNA. Methods to transfect cells with mRNA include electroporation, chemical transfection methods such as lipofection or cationic polymers. As another example, the gene delivery vector may be a viral vector derived from a virus, *e*.*g*., an adenovirus, an adeno-associated virus, a LV, a herpes virus, an alphavirus or a retrovirus, *e*.*g*., Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) or Rous Sarcoma Virus (RSV). While embodiments encompassing the use of LV are described in greater detail below, it is expected that the ordinarily skilled artisan will appreciate that similar knowledge and skill in the art can be brought to bear on non-LV gene therapy vectors as well. In particular, viral vectors include retroviral vectors, for example for stable gene expression in target cells; and adenoviral or adeno-associated viral vectors for transient gene expression in target cells. Retroviral vectors include gamma-retroviruses, lentiviruses, NILV-S/MAR vectors (using non-integrating lentiviral (NILV) vector containing a scaffold/matrix attachment region (S/MAR) element). Nonviral vectors include mRNA vectors, transposons and lipid nanoparticles. Vectors can also comprise sequences encoding proteins for the expression of a transgene or silencing of target genes, such as transposase enzymes (*e*.*g*., sleeping beauty, piggyBac); and endonuclease enzymes, for example zinc finger nucleases, TALENs, CRISPR/Cas9. Methods for vector and transgene delivery include electroporation, lipid nanoparticles, nucleofection, hydrodynamic delivery, and cell-penetrating peptides.

Gamma-retroviruses and lentiviruses are most often used in manufacturing CAR T cells due to their high transduction efficiencies. In a preferred aspect, a vector as described herein is a retroviral vector, preferably a lentiviral vector (LV) comprising a polynucleotide encoding PHGDH as described herein, preferably an LV expression vector comprising an expression cassette comprising a polynucleotide encoding PHGDH as described herein. The LV expression vector can be a self-limiting LV expression vector, a self-inactivating LV expression vector or a non-integrating LV expression vector.

A "lentivirus" as used herein refers to a genus of the Retroviridae family. Lentiviruses are unique among the retroviruses in being able to infect non-dividing cells; they can deliver a significant amount of genetic information into the DNA of the host cell, so they are one of the most efficient methods of vector gene delivery. HIV, SIV, and FIV are all examples of lentiviruses. Vectors derived from lentiviruses offer the means to achieve significant levels of gene transfer *in vivo.* LV vectors are well characterized and their production is known in the art.

One or more LV vectors are used to produce an LV expression vector comprising an expression cassette comprising a polynucleotide encoding a gene of interest. The following

LV plasmid vectors are typically used to produce an LV expression vector: 1) a transfer plasmid comprising a polynucleotide encoding a gene of interest, 2) one or more packaging plasmid(s), and 3) an envelope plasmid. LV plasmid vectors are then transfected into cells, preferably *in vitro* (for example in HEK-293 cells), to generate an LV expression vector comprising an expression cassette that can be used to transduce cells to express the gene of interest (*i*.*e*., LV particles comprising an expression cassette that can be used to transduce cells to express the gene of interest).

A vector of the invention can comprise any LV plasmid vector as described herein or combination thereof. An LV plasmid vector of the invention can comprise a transfer plasmid comprising a polynucleotide encoding PHGDH as described herein. The transfer plasmid can be any of the transfer plasmids known in the art and as described herein. Preferably, the transfer plasmid is an HIV-1 replication incompetent transfer plasmid, preferably selected from a 2^{nd} or 3^{rd} generation transfer plasmid, preferably a 3^{rd} generation transfer plasmid, preferably selected from pLV-mcherry and pSFFV. Packaging and envelope plasmids can be selected from any of the plasmids known in the art and as described herein for use with the transfer plasmid described herein and based on their compatibility. For example, a 2^{nd} generation transfer plasmid must be used with a 2^{nd} generation packaging plasmid. A 3^{rd} generation transfer plasmid can be used with a 2^{nd} generation or 3^{rd} generation packaging plasmid. In a preferred aspect, the packaging plasmid is a 2^{nd} generation packaging plasmid, preferably selected from psPAX2 or pCMVR.8.74. Envelope plasmids are interchangeable and typically encode for VSV-G. In a preferred aspect, the envelope plasmid is pMD2G or pCMV.

In one aspect, the disclosure provides a kit comprising one or more of the vectors described herein, preferably an LV vector as described herein, preferably a transfer plasmid as described herein and one or more LV vector as described herein selected form one or more packaging plasmid as described herein and/or an envelope plasmid as described herein.

In preferred aspects, the disclosure relates to a vector as described herein, preferably an LV expression vector, comprising an expression cassette comprising a polynucleotide sequence encoding PHGDH and a polynucleotide sequence encoding a CAR as described herein.

In an alternative aspect, the disclosure relates to a vector as described herein, preferably an LV expression vector, comprising an expression cassette comprising a polynucleotide sequence encoding PHGDH as described herein and a separate vector comprising an expression cassette comprising a polynucleotide sequence encoding a CAR as described herein.

A vector as disclosed herein can also be used to introduce a polynucleotide sequence or polypeptide that increases the expression of endogenous PHGDH. For example, the activity of one or more endogenous promoter and/or enhancer regions associated with the PHGDH gene can be increased by methods known in the art. Silencing of endogenous PHGDH expression can be prevented or reduced, for example by introducing a polynucleotide sequence encoding an siRNA, antisense, ribozyme, shRNA, methylation of CpG nucleotides of the PHGDH gene within the promotor region, non-coding RNA-associated gene silencing.

The disclosure also provides a cell, for example one or more cells, comprising any of the polynucleotide sequences, expression cassettes, vectors and/or expression vectors as disclosed herein. The cell can be any cell useful in recombinant genetic techniques, for example for preparing a polynucleotide sequence, expression cassette, vector, expression vector and/or cell as described herein. In a preferred aspect, the cell can be any cell for producing an expression vector, preferably an LV expression vector, comprising an expression cassette that can be used to transduce cells to express PHGDH (*i*.*e*., LV particles comprising an expression cassette that can be used to transduce cells to express PHGDH). The cell can be selected from a mammalian cell (*e.g*., 293 cells), an insect cell (*e.g*., SF9 cells), a microorganism and a yeast. The cells can be packaging cells in which LV vector genes are stably maintained or producer cells in which the LV vector genome is stably maintained and packaged. Exemplary packaging and producer cells are derived from SF-9, 293, A549 or HeLa cells. LV vectors can be purified and formulated using standard techniques known in the art. The cell can be transduced with a viral vector comprising an expression cassette disclosed herein or can have an expression cassette disclosed herein integrated into the cell's genome. In certain embodiments, the cell is a cell used to produce a viral gene delivery vector (*e*.*g*., LV particles comprising an expression cassette that can be used to transduce cells to express PHGDH). In other embodiments, the cell is any cell useful in regenerative medicine, for example a cellular therapy as described herein. Suitably, the cell is for delivery to a subject.

The disclosure also provides a composition comprising any of the polynucleotide sequences, expression cassettes, vectors, expression vectors and/or cells as disclosed herein. In a preferred aspect, the composition can be a pharmaceutical composition suitably comprising one or more pharmaceutically acceptable carrier, diluent and/or excipient. Compositions can comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (*e*.*g*., aluminum hydroxide); and preservatives.

The polynucleotides, expression cassettes, vectors and compositions disclosed herein, can be used in a method for enforcing the expression of PHGDH in a cell. The polynucleotides, expression cassettes, vectors and compositions can be used in a method for expressing a PHGDH transgene in a cell as disclosed herein. In one aspect, the invention provides a method of producing a cell genetically engineered to enforce expression of PHGDH comprising contacting one or more cells with a polynucleotide, expression cassette, vector or composition as disclosed herein. The method can be a research method, *e*.*g*. to determine the effect PHGDH has on cell viability and/or function, or a medical method, *e*.*g*. to treat or prevent a disease or disorder as disclosed herein or to prepare a cell or composition as disclosed herein for treating or preventing a disease or disorder as disclosed herein. In some embodiments, contacting occurs *in vitro* or *ex vivo.* In some aspects, contacting occurs *in vivo, i.e*., the polynucleotide, expression cassette, vector or composition as disclosed herein can be administered to a subject. In a preferred aspect, the contacting occurs *in vitro* or *ex vivo.*

In a preferred aspect, the one or more cells can be any cells useful in regenerative medicine, preferably a cellular therapy as disclosed herein, preferably an adoptive cell therapy, preferably a CAR T cell therapy. In this aspect, the cells, preferably cells genetically engineered to enforce expression of PHGDH or a composition comprising such cells, can be administered to a subject in need thereof. In a preferred aspect, the one or more cells are immune cells, preferably lymphocytes, preferably T cells, preferably CD3⁺ cells, preferably CD8⁺ T cells and/or CD4⁺ T cells, for example bulk T cells comprising CD8⁺ T cells and CD4⁺ T cells.

The cells can be obtained from a subject. In a preferred aspect, the cells can be obtained from a subject to be treated by the cellular therapy (autologous), for example the cells can be obtained from the subject prior to the cellular therapy. In other aspects, the cells can be obtained from a subject other than the subject to be treated by the cellular therapy (*e*.*g*., allogeneic from a donor). Preferably, the cell is a mammalian cell, preferably a human cell.

Cells as described herein can be obtained from a subject from any source using methods known in the art. In a preferred aspect, immune cells as disclosed herein, preferably one or more lymphocytes, can be obtained from a subject from a number of sources, including peripheral blood, mononuclear cells, bone marrow, lymph node tissue, blood, thymus tissue, tissue from a site of infection, ascites, pleural effusion, spleen tissue, and tumors. Any type of lymphocyte available in the art and described herein may be used. In general, the skilled person is aware of methods to isolate a certain type of lymphocyte from a suitable source. In a preferred aspect, the method comprises obtaining peripheral blood mononuclear cells (PBMCs) from a subject by methods known in the art, preferably from the blood. Preferably PBMCs are non-mobilized. PBMCs can be obtained from a blood sample from the subject by methods known in the art, for example by density-gradient centrifugation protocols (Lymphoprep; Fresenius Kabi) by Ficoll^{™} separation or by apheresis, preferably leukapheresis. Cells obtained by apheresis may be washed to remove the plasma fraction and to place the cells in an appropriate buffer or media for subsequent processing steps. In a preferred aspect, T cells can be obtained from PBMCs by methods known in the art.

*Ex vivo* or *in vitro* cellular therapy methods are known in the art and include one or more of isolating, enriching, activating and expanding cells. One or more *ex vivo* cellular therapy methods can be carried out after obtaining the cells from the subject and/or prior to administering the cells to the subject.

Cells can be isolated and/or enriched by positive or negative selection techniques known in the art and as described herein, such as using density-gradient centrifugation and/or magnetic beads. In the methods described herein, isolation and/or enrichment can occur prior to or after contacting the one or more cells with a polynucleotide, expression cassette, vector or composition as disclosed herein. Lymphocytes have been well characterized and it is known in the art what surface antigens can be present on different types of lymphocyte. The skilled person is capable of selecting conditions for positive or negative selection that allow for the enrichment or isolation of specific types of cell, in particular specific types of lymphocyte. In addition, commercial kits are readily available for the enrichment and/or isolation of specific cells, in particular lymphocytes. In a preferred aspect, the method comprises isolating and/or enriching for a subpopulation of lymphocytes, such as CD3⁺, CD28⁺, CD4⁺, CD8⁺, CD45RA⁺, and CD45RO⁺ T cells, CD16⁺ and CD56⁺ NK cells or CD3⁺, CD56⁺ and CD161⁺ NKT cells; preferably CD3⁺cells, preferably CD8⁺ cells and/or CD4⁺ cells, preferably CD8⁺ cells.

Cells can be activated and/or expanded before they are administered to a subject by techniques known in the art and as described herein, for example using antibody coated micro beads, such as anti-CD3/anti-CD28 coated microbeads. In the methods described herein, activation and/or expansion can occur prior to or after contacting the one or more cells with a polynucleotide, expression cassette, vector or composition as disclosed herein. Activation and/or expansion can occur prior to or after isolation and/or enrichment. Cells can also be activated and/or expanded after they have been administered to a subject by techniques known in the art.

NK cells described herein can be activated by culturing the cells in appropriate medium (e.g. Minimal Essential Media or RPMI Media 1640 or, X-vivo 15, (Lonza), CellGro media (Cellgenix), IMDM (Gibco)) that may contain factors necessary for proliferation and viability, including serum (e.g., fetal bovine, human or horse serum) supplemented with IL-15 and/ or IL-12 and/ or IL-18. Activation of NK cells can also be accomplished by the supplementation of the media with IL-2. Activation of NK cells may be improved by adding a feeder cell line to the culture.

T cells described herein can be activated by contact with a surface having attached thereto an agent that stimulates a CD3/TCR complex associated signal and a ligand that stimulates a co-stimulatory molecule on the surface of the T cells. In particular, T cell populations may be stimulated by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule may be used. For example, a population of T cells may be contacted with an anti-CD3 antibody and an anti-CD28 antibody, under conditions appropriate for stimulating proliferation of the T cells.

In a preferred aspect, the method as described herein comprises obtaining cells from a subject by leukapheresis of non-mobilized peripheral blood mononuclear cells, enriching and selecting T cells expressing CD3, preferably T cells expressing CD8 and CD4, *ex vivo,* and activating the T cells using anti-CD3 and/or anti-CD28 antibodies in the presence of IL2.

The invention provides one or more cells as disclosed herein transduced with a vector comprising a polynucleotide encoding PHGDH and optionally a polynucleotide encoding a CAR as disclosed herein; or one or more cells as disclosed herein transduced with a vector comprising a polynucleotide encoding PHGDH and optionally a separate vector comprising a polynucleotide encoding a CAR as disclosed herein. The one or more cells can comprise a control switch, such as a suicide switch, endogenous switch or exogenous switch. Accordingly, the present invention provides a method of transducing one or more cells as disclosed herein with a vector comprising a polynucleotide encoding PHGDH and optionally a polynucleotide encoding a CAR as disclosed herein; or a vector comprising a polynucleotide encoding PHGDH and a separate vector comprising a polynucleotide encoding a CAR as disclosed herein. In various aspects, the method as described herein can comprise isolation and/or enrichment after contacting the one or more cells with a polynucleotide, expression cassette, vector or composition as disclosed herein. In one aspect, the method comprises isolation and/or enrichment of cells transduced with a vector comprising a polynucleotide encoding PHGDH, and optionally a polynucleotide encoding a CAR as disclosed herein; or isolation and/or enrichment of cells transduced with a vector comprising a polynucleotide encoding PHGDH and optionally a separate vector comprising a polynucleotide encoding a CAR as disclosed herein.

The term "transduced" or "transfected" as used herein refers to a process by which exogenous nucleic acid is transferred or introduced into the host cell. A "transfected" or "transduced" cell is one which has been transfected or transduced with exogenous nucleic acid.

The disclosure also provides a method of treatment comprising administering any of the cells as disclosed herein or any of the compositions, polynucleotide sequences, expression cassettes, vectors and/or expression vectors disclosed herein to a subject in need thereof. In a preferred aspect, the method of treatment comprises any treatment where enhanced persistence of cells is needed, for example any treatment in the field of regenerative medicine, preferably a cellular therapy as described herein, preferably an adoptive cell therapy as described herein.

The disclosure also provides any of the cells as disclosed herein or any of the compositions, polynucleotide sequences, expression cassettes, vectors and/or expression vectors disclosed herein for use in any of the methods described herein, in particular for use in any of the methods of treatment described herein.

The disclosure also provides the use of any of the cells as disclosed herein or any of the compositions, polynucleotide sequences, expression cassettes, vectors and/or expression vectors disclosed herein in the manufacture of a medicament for any of the methods described herein, in particular for a method of treatment as described herein.

The terms "treating," "treatment", and the like are used herein to generally mean obtaining a desired pharmacologic and/or physiologic effect. The effect may be prophylactic in terms of completely or partially preventing a disease or condition and/or may be therapeutic in terms of a partial or complete cure for a disease or condition and/or adverse effect attributable to the disease. "Treatment" as used herein covers any treatment of a disease or condition in a mammal, preferably a human, and includes: ameliorating a disease, disorder or condition (*i*.*e*., slowing or arresting or reducing the development of the disease, disorder or condition or at least one of the clinical symptoms thereof); alleviating or ameliorating at least one physical parameter including those which may not be discernible by the patient; modulating the disease, disorder or condition, either physically, (*e*.*g*., stabilization of a discernible symptom), physiologically, (*e*.*g*., stabilization of a physical parameter), or both; or preventing or delaying the onset or development or progression of the disease, disorder or condition or one or more clinical symptoms thereof.

As used herein, the phrase "ameliorating at least one symptom of" refers to decreasing one or more symptoms of the disease or condition for which the subject is being treated. The disease or condition being treated can be selected from any of the diseases or conditions disclosed herein.

In a preferred aspect, the invention provides an improved cellular therapy and polynucleotides, expression vectors and methods for preparing and using the same. In particular, the invention provides an improved cellular therapy, preferably any of the cellular therapies disclosed herein, comprising one or more cells as described herein that are transplanted into a subject suffering from a disease or condition in order to treat the disease or condition, wherein the invention improves the persistence and/or survival of the transplanted cells. In a preferred aspect, the invention provides an improved adoptive cell therapy, wherein the one or more transplanted cells are immune cells, preferably lymphocytes, preferably T cells as described herein, optionally comprising one or more CAR as described herein. In a preferred aspect, the invention provides an improved CAR T cell therapy, in particular a CAR T cell therapy as described herein. In a preferred aspect, a method of treating any of the diseases or conditions as described herein can comprise administering any of the cells or pharmaceutical compositions as described herein to a subject in need thereof.

Several cellular therapies have received regulatory approval for the treatment of various diseases and conditions. CAR T cell therapies have been approved for treating diseases including cancer, in particular hematologic or blood cancers. Hematopoietic progenitor cell (HPC) transplantation (stem cell or bone marrow transplant, also known as HSCT) is approved for treating diseases including amyloidosis, Germ cell tumors (*e*.*g*., testicular cancer), Acute leukemia, Amegakaryocytosis or congenital thrombocytopenia, Aplastic anemia or refractory anemia, Chronic lymphocytic leukemia, Familial erythrophagocytic lymphohistiocytosis, Myelodysplastic syndrome of another myelodysplastic disorder, Osteopetrosis, Paroxysmal nocturnal hemoglobinuria, and Wiskott-Aldrich syndrome. HSCT can be autologous or allogeneic, such as salvage allogeneic HSCT. Other approved cellular therapies include treatments for melanoma, prostate cancer, mucogingival conditions, retinal conditions and diseases, cartilage defects, athymia, burns and muscular atrophy.

Approved CAR T cell therapies include:
Idecabtagene vicleucel (Tradename: ABECMA; STN: BLA 125736; Celgene Corporation) is approved for treatment of adult patients with relapsed or refractory multiple myeloma after four or more prior lines of therapy including an immunomodulatory agent, a proteasome inhibitor, and an anti-CD38 monoclonal antibody.

Lisocabtagene maraleucel (Tradename: BREYANZI; STN# BLA 125714; Juno Therapeutics, Inc.) is approved for the treatment of adult patients with relapsed or refractory large B-cell lymphoma after two or more lines of systemic therapy, including diffuse large B-cell lymphoma (DLBCL) not otherwise specified (including DLBCL arising from indolent lymphoma), high-grade B-cell lymphoma, primary mediastinal large B-cell lymphoma, and follicular lymphoma grade 3B.

Tisagenlecleucel (Tradename: KYMRIAH; STN: 125646; Novartis) is approved for the treatment of pediatric and young adult patients (age 3-25 years) with B-cell precursor acute lymphoblastic leukemia (ALL) that is refractory or in second or later relapse. Adult patients with relapsed or refractory (r/r) large B-cell lymphoma after two or more lines of systemic therapy including diffuse large B-cell lymphoma (DLBCL) not otherwise specified, high grade B-cell lymphoma and DLBCL arising from follicular lymphoma.

Brexucabtagene autoleucel (Tradename: TECARTUS; STN: BL 125703; Kite Pharma, Inc.) is approved for treating adult patients with relapsed or refractory mantle cell lymphoma (MCL). New Indication for this supplement: Adult patients with relapsed or refractory (r/r) B-cell precursor acute lymphoblastic leukemia (ALL).

Axicabtagene ciloleucel (Trade Name: YESCARTA; STN: BL 125643; Kite Pharma Inc.) is approved for the treatment of adult patients with relapsed or refractory large B-cell lymphoma after two or more lines of systemic therapy, including diffuse large B-cell lymphoma (DLBCL) not otherwise specified, primary mediastinal large B-cell lymphoma, high grade B-cell lymphoma, and DLBCL arising from follicular lymphoma. Axicabtagene ciloleucel is not indicated for the treatment of patients with primary central nervous system lymphoma.

The terms "patient," "subject," "individual," and the like are used interchangeably herein, and refer to any animal, or cells thereof whether in vitro or in situ, amenable to the methods described herein. In certain non-limiting embodiments, the patient, subject or individual is a human.

In a preferred aspect, a method of treating a disease or condition as described herein can comprise treating cancer. The term "cancer", as used herein is as disease characterized by the rapid and uncontrolled growth of aberrant cells. Cancer cells can spread locally or through the bloodstream and lymphatic system to other parts of the body.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myclogonous leukemia, and chronic lymphocytic leukemia), polycythemia vera, lymphoma, Hodgkin's disease, non-Hodgkin's lymphoma (indolent and high grade forms), mantle cell lymphoma, multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors include fibrosarcoma, myosarcoma, liposarcoma, chondrosarcoma, osteosarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (such as a glioma (such as brainstem glioma and mixed gliomas), glioblastoma (also known as glioblastoma multiforme) astrocytoma, CNS lymphoma, germinoma, medulloblastoma, Schwannoma craniopharyogioma, ependymoma, pinealoma, hemangioblastoma, acoustic neuroma, oligodendroglioma, menangioma, neuroblastoma, retinoblastoma and brain metastases).

The cancer can be a hematologic cancer as described herein, preferably selected from acute lymphoblastic leukemia, diffuse large B-cell lymphoma, Hodgkin's lymphoma, acute myeloid leukemia, multiple myeloma, and mantle cell lymphoma; or a solid tumor, preferably selected from colon cancer, breast cancer, pancreatic cancer, ovarian cancer, hepatocellular carcinoma, lung cancer, neuroblastoma, glioblastoma or sarcoma.

In a preferred aspect, the method of treating a disease or condition as described herein can comprise administering any of the cells or pharmaceutical compositions as described herein to a subject, in particular a cell genetically engineered to enforce expression of PHGDH as described herein, preferably a T cell, preferably wherein the cell comprises a CAR as disclosed herein or a modified TCR. In one aspect, the CAR or TCR can comprise one or more antigen binding domain (ABD) that specifically binds to one or more of BCMA, CD19, CD22, CD30, CD33, CD56, CD123, CEA, EBV-related antigens, EGFR, GD2, GPC3, HER2, HPV-related antigens, MAGE antigens, Mesothelin, MUC-1, NY-ESO-1, PSCA, PSMA, ROR1, WT1, and Claudin 18.2; preferably one or more of CD19, BCMA, CD30, CD33, CD123 or FLT3; preferably an ABD that specifically binds to CD19 or BCMA.

In one aspect, the CAR or TCR can comprise a CD19 ABD and the method comprises treating a cancer arising from B cells, preferably a B cell lymphoma, acute lymphoblastic leukaemia (ALL), or chronic lymphocytic leukaemia (CLL). In a preferred aspect, the method comprises treating adult patients with relapsed or refractory (r/r) large B-cell lymphoma, including diffuse large B-cell lymphoma (DLBCL), (DLBCL) not otherwise specified, high grade B-cell lymphoma and DLBCL arising from follicular lymphoma, DLBCL arising from indolent lymphoma, high-grade B-cell lymphoma, primary mediastinal large B-cell lymphoma, and follicular lymphoma grade 3B; pediatric and young adult patients (age 3-25 years) with B-cell precursor acute lymphoblastic leukemia (ALL), which can be refractory or in second or later relapse; adult patients with relapsed or refractory mantle cell lymphoma (MCL); Adult patients with r/r ALL.

In another aspect, the CAR or TCR can comprise a BCMA ABD and the method comprises treating a blood cancer, preferably multiple myeloma. In a preferred aspect, the method comprises treating adult patients with relapsed or refractory multiple myeloma, such as after four or more prior lines of therapy including an immunomodulatory agent, a proteasome inhibitor, and an anti-CD38 monoclonal antibody.

In one aspect, a method of treating a disease or condition as described herein can comprise treating an infection in a subject, such as a viral infection or fungal infection, preferably a viral infection. In a preferred aspect, the method of treating a disease or condition as described herein can comprise administering any of the cells or pharmaceutical compositions as described herein, in particular a cell genetically engineered to enforce expression of PHGDH as described herein, preferably a T cell, preferably wherein the cell comprises a CAR as disclosed herein or a modified TCR. In one aspect, the CAR or TCR can comprise one or more ABD that specifically binds to one or more viral antigen. The viral antigen may be any component of a virus particle that is accessible to the CAR or TCR, for example an antigen that forms part of the surface and/or the protein coat of the virus. Preferably, the viral antigen is an antigen derived from human immunodeficiency virus (HIV), adenoviruses, polyomaviruses, influenza virus, hepatitis B virus (HBV), or human herpesvirus, in particular wherein the human herpesvirus is cytomegalovirus (CMV), Epstein-Barr virus (EBV), herpes simplex virus (HSV), Varizella-Zoster virus (VZV) or human herpesvirus 8 (HHV8).

In a preferred aspect, the method of treating a disease or condition as described herein can comprise administering any of the cells or pharmaceutical compositions comprising the cells as described herein to a subject as part of a cellular therapy. The administering of any of the cells or pharmaceutical compositions comprising the cells as described herein to a subject as part of a cellular therapy may be referred to as cell transplanting or grafting herein. In particular, a cell genetically engineered to enforce expression of PHGDH as described herein, preferably an immune cell, preferably a T cell, preferably wherein the cell comprises a CAR as disclosed herein or a modified TCR can be administered to a subject as part of a cellular therapy. The cells can be autologous or allogeneic, preferably autologous. The method of treating a disease or condition can comprise obtaining the cells from the subject, for example the method can comprise obtaining PBMCs from the subject by methods known in the art and as described herein, preferably by leukapheresis of non-mobilized PBMCs and PBMC isolation. The method can further comprise any of the *ex vivo* or *in vitro* cellular therapy methods known in the art and described herein, include one or more of isolating, enriching, activating and expanding cells as disclosed herein. The method can comprise a method of activating and/or expanding cells *in vivo* by methods known in the art and as disclosed herein. One or more *ex vivo* or *in vitro* cellular therapy method can be carried out after obtaining the cells from the subject and/or prior to administering the cells to the subject. In a preferred aspect, the method comprises isolating and/or enriching for a subpopulation of lymphocytes, preferably T cells, preferably CD3⁺ cells, preferably CD8⁺ and/or CD4⁺ cells, preferably CD8⁺ cells. In a preferred aspect, the method comprises activating the cells, preferably T cells, using anti-CD3 and/or anti-CD28 antibodies.

The method of treating a disease or condition as described herein can comprise administering any of the cells as disclosed herein or any of the compositions, polynucleotide sequences, expression cassettes, vectors and/or expression vectors disclosed herein to a subject in need thereof in combination with one or more other therapeutic agent or modality. Compositions described herein can comprise an effective amount of the cells as disclosed herein or any of the compositions, polynucleotide sequences, expression cassettes, vectors and/or expression vectors disclosed herein in combination with one or more other therapeutic agent or modality. Other therapeutic agents or modalities can be selected from other known treatments for the diseases disclosed herein.

All of the features described herein (including any accompanying claims, abstract and drawings), and/or all of the steps of any method so disclosed, may be combined with any of the above aspects in any combination, except combinations where at least some of such features and/or steps are mutually exclusive. Specifically, any of the active agents and compositions described herein can be used in any of the described methods of treatment. Any and all such combinations are explicitly envisaged as forming part of the invention.

### Examples

### Example 1 - Metabolic pruning is a hallmark of memory T cell differentiation

The focus of this study was the comparison of metabolic programs between CD8⁺ T_{NV} and T_{EM}. Specifically, CD8⁺ T cells were isolated from buffy coats from healthy donors and sorted by flow cytometry followed by RNAseq and proteomics (Figure 1A). *'Cellular metabolic process'* was the most significantly enriched GO term among downregulated RNA species in CD8⁺ T_{EM} vs. T_{NV} cells (Figure 1B). At the transcript and protein level, PHGDH was the most downregulated metabolic transcript in T_{EM} cells, and one of the most differentially expressed proteins overall (Figure 1C). Differential expression of PHGDH between CD8⁺ T_{EM} and T_{NV} was confirmed by Western blotting (Figure 1D).

Naïve murine T cells induce the components of the serine synthesis pathway (SSP) upon activation, *i*.*e*. in primary effector cells (FIGURE 1E). This led us to test whether in secondary effector cells, *i*.*e*. those derived from CD8⁺ T_{CM} and T_{EM} cells, expression of PHGDH, PSAT1 and PSPH was also upregulated. Following stimulation with anti-CD3/CD28, only a slight induction of PHGDH was detected in T_{EM} cells and T_{CM} cells (Figure 1F) compared to T_{NV} cells. In T_{NV} cells, abundance of PHGDH increased further (Figure 1F).

Given that CD8⁺ T_{CM} and T_{EM} cells did not express PHGDH, we reasoned that extracellular serine might also selectively affect their capacity to proliferate. Indeed, in absence of serine both activated T_{CM} and T_{EM} failed to proliferate, whereas proliferation of T_{NV} cells was less affected (Figure 1G).

These data identified selective silencing of PHGDH expression in CD8⁺ T_{EM} cells. This trait was maintained when these cells were restimulated and became secondary effectors. Further, T_{EM} cells strictly depend on exogenous serine for proliferation, thus being serine auxotroph.

### Example 2 - PHGDH is an RNA binding protein

To explore the notion of a moonlighting role of PHGDH its structure was compared to the non-canonical RNA-binding proteins (RBP) GAPDH; both being NAD⁺-dependent dehydrogenases, *in silico.* For GAPDH, several regions have been proposed to bind RNA, including the Rossmann fold (a super-secondary structure responsible for interaction with the di-nucleotide NAD⁺), the positively charged substrate binding groove, and the dimer interface. Several similarities between the two enzymes were noted: beside the Rossmann fold, PHGDH also displayed a positively charged substrate groove and a dimer interface with basic and aromatic residues (Figure 2A).

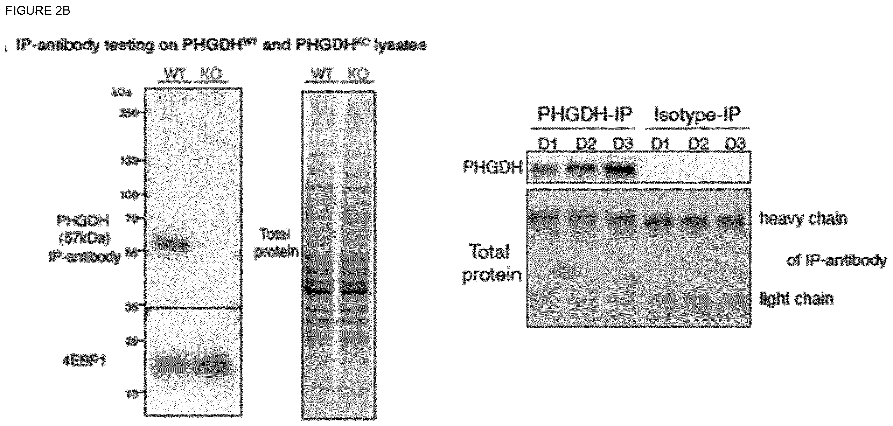

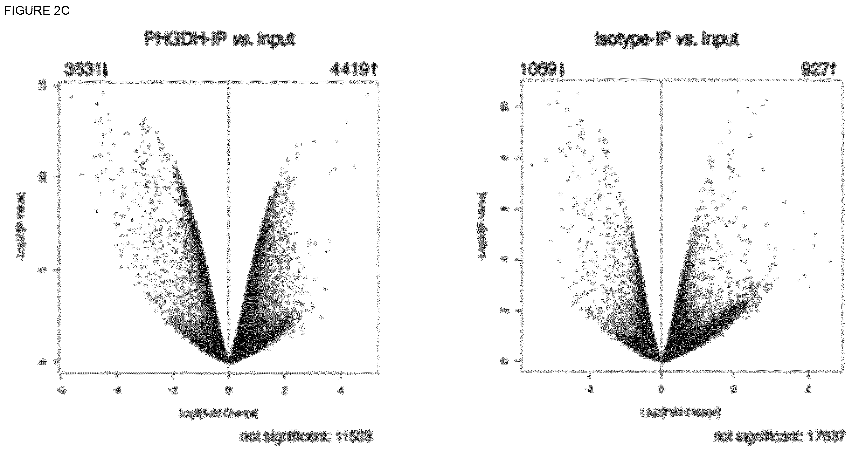

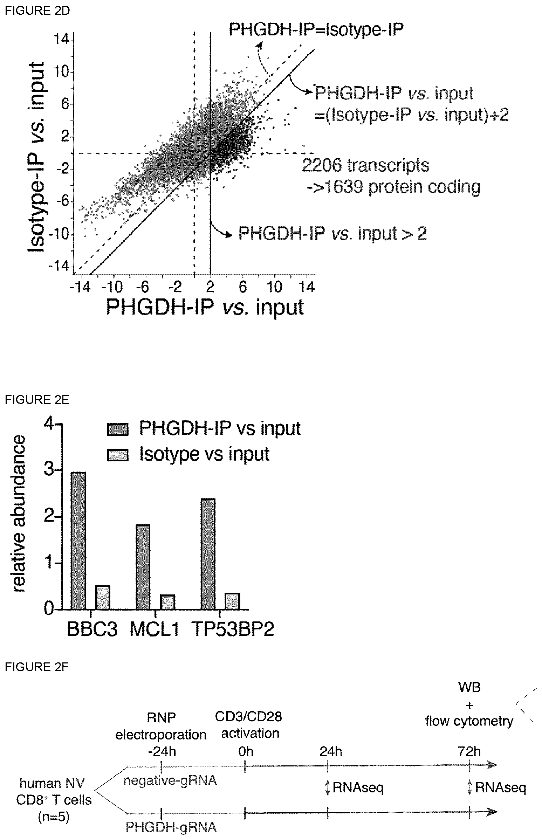

To directly explore a potential RNA-binding role of PHGDH in T cells, RNA immunoprecipitation sequencing (RIPseq) experiments were performed with primary human effector T cells. Specificity of the PHGDH-immunoprecipitation (IP) antibody was validated using lysates from PHGDH^{KO} Jurkat T cells (Figure 2B, left panel). In addition, IP of PHGDH was confirmed for each sample before sequencing of co-precipitated RNA, isotype matched antibodies served as control (Figure 2B, right panel). Compared to input samples, PHGDH-IP significantly enriched 4419 transcripts, whereas isotype-IP enriched 927 (Figure 2C). To visualize the filtering strategy, transcripts enriched by isotype-IP over input material were plotted against transcripts enriched in the PHGDH-IP over input material. RNA species that were (i) enriched >2 fold in the PHGDH-IP over input, and (ii) enriched 2-times more in the PHGDH-IP when compared to the isotype-IP, were considered to specifically interact with PHGDH. This filtering process selected for 1639 protein coding transcripts (Figure 2D), suggesting RNA-binding features of PHGDH, *i*.*e*. functional features of an RBP, in primary human T cells. Since expression of PHGDH is a hallmark of undifferentiated cells (data not shown), it was speculated that it might be relevant to maintain survival through its RNA-binding properties. Remarkably, pro-survival genes, namely *BBC3*, *MCL1* and *TP53BP2* were detected among PHGDH bound RNA species in RIPseq experiments (Figure 2E).

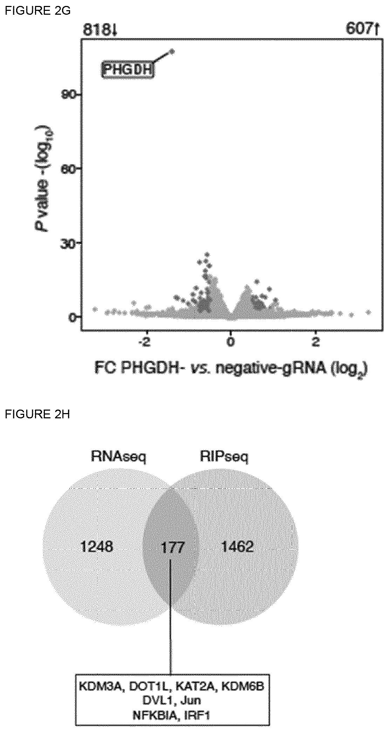

To further explore this notion, PHGDH knockout experiments were designed with sorted primary human T_{NV} cells using CRISPR/CAS9 (Figure 2F). To ensure that loss of PHGDH predominantly affected the enzymes' RNA-binding properties, and to prevent a metabolic stress response, experiments were carried out in presence of abundant extracellular serine. Three days post-activation PHGDH mRNA was the most downregulated transcript among 1425 differentially expressed transcripts/genes (DEGs) (Figure 2G) with their small fold-changes aligning with post-transcriptional interference described for RBPs (Lu et al., 2014, Cell Rep. 9, 2330-2343; Díaz-Muñoz et al., 2015, Nature Immunology. 16, 415-425; and Monzón-Casanova et al., 2020, eLife. 9, 613-28). Among these DEGs, roughly 10% (177) were specifically pulled-down also in the RIPseq experiment, including RNA species coding for epigenetic modifiers (KDM3A, DOT1L, KAT2A and KDM6B), components of WNT signaling (DVL1 and Jun) and transcripts impacting survival (NFKBIA and IRF1) (Figure 2H).

Together, these data suggested that PHGDH is moonlighting as an RBP, and the findings were compatible with the notion that, through RNA-binding, PHGDH may contribute to regulating T cell differentiation or survival.

### Example 3 - Enforced expression of PHGDH enhances T cell persistence in vivo

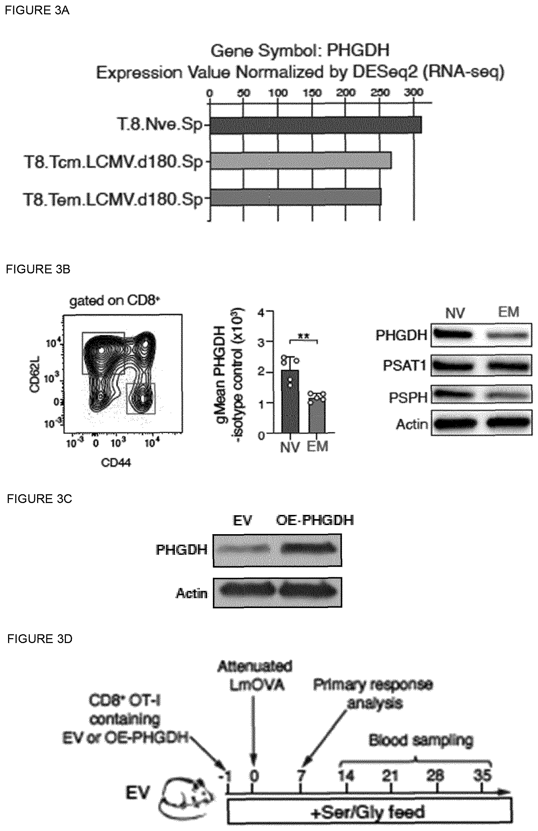

To further explore the role of PHGDH in regulating T cell survival, we established an *in vivo* murine infection model. Analysis of RNAseq data from the ImmGen Consortium (immgen.org) suggested that the expression pattern of PHGDH between murine CD8⁺ naïve, central memory and effector memory T cells is similar to human CD8⁺ T cells (Figure 3A). PHGDH expression levels were further analysed in murine T_{NV} and T_{EM} CD8 T cells by Western blot and FACS. Those experiments confirmed decreased expression of PHGDH in memory cells (Figure 3B). To test the *in vivo* relevance, ovalbumin-specific CD8⁺ T cells (OT-I) overexpressing PHGDH (OE) or as a control an empty vector (EV) were generated (Figure 3C). Overexpression was enforced by retroviral transduction. Plasmid constructs were: MigR1-PHGDH-IRES-EGFP (OE-PHGDH), and MigR1-IRES-EGFP (EV) vectors (addgene).

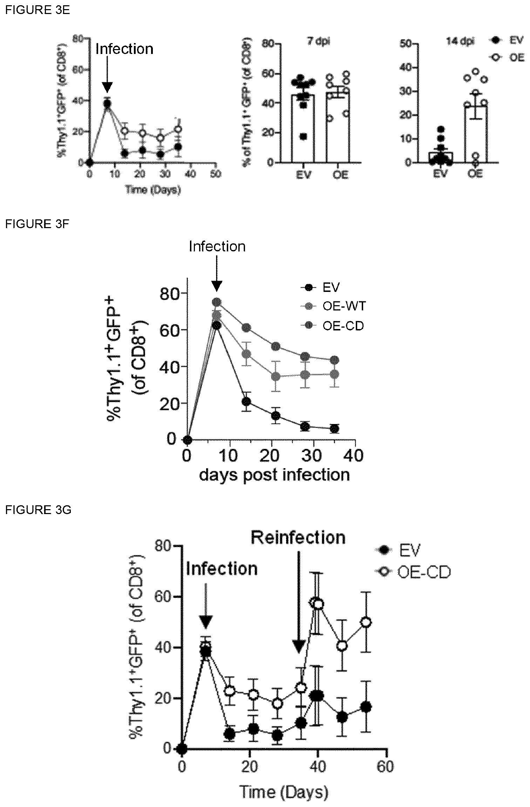

To examine whether overexpression of PHGDH affected expansion or survival of OT-I CD8⁺ T cells, OE or EV OT-I cells were adoptively transferred into C57BL/6 recipients (FIGURE 3D). One day post transfer, recipient mice were infected with LmOVA and expansion of transferred T cells in blood was tracked. At peak of expansion (7 dpi), OE and EV OT-I cells were detected at comparable levels (Figure 3E). However, OE-PHGDH OT-I T cells did not contract to the same extent as control cells, resulting in higher frequencies at 14, 21, 28 and 35 dpi (Figure 3E).

In order to dissect if the canonical or potential mRNA binding ability of PHGDH resulted in the observed phenotype, OT-I T cells overexpressing the wild-type (OE-WT) or catalytic dead (OE-CD) variant of PHGDH were generated. OE-WT, OE-CD or EV OT-I T cells were transferred into recipients that were then infected with LmOVA. Similar to the previous experiment, frequencies of transferred cells 7 dpi were comparable but resulted in different numbers at later time points. As before, PHGDH overexpressing cells were detected at much higher frequencies and, remarkably, OE-CD OT-I T cells persisted at even increased numbers when compared to OE-WT OT-I T cells (Figure 3F). To test if the OE-CD OT-I T cells could respond to a second stimuli, we rechallenged recipients with LmOVA. No impact of increased persistence was observed on OE-CD OT-I cells' ability to re-expand (Figure 3G). These data provide evidence that preventing the silencing of PHGDH in the phase of effector to memory transition can increase persistence of T cells.

### Example 4- Enforced expression of PHGDH augments in vitro and in vivo persistence of T cells

Building on the previous data, we reasoned that overexpression of PHGDH may improve persistence and thus the therapeutic potential of human (CAR) T cells.

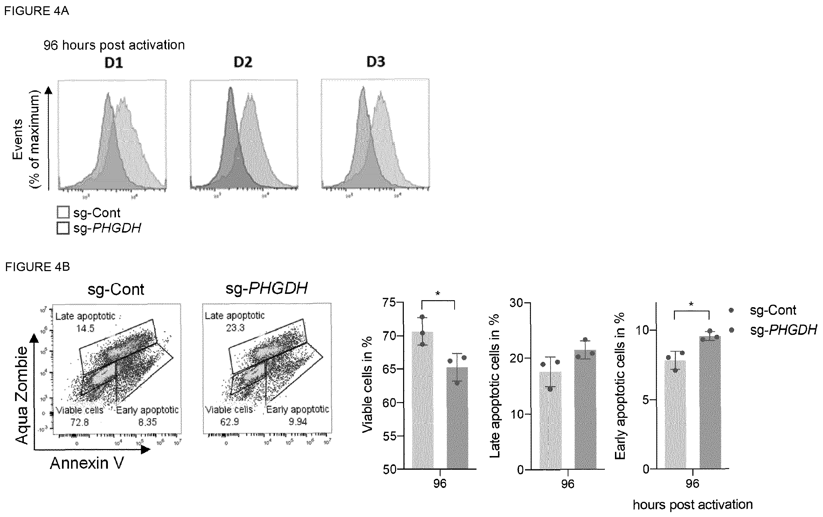

To further examine the role of PHGDH in regulating persistence of T cells, PHGDH was knocked-out in human naive CD8⁺ T cells using CRISPR/Cas and cells were activated 24 hours later using anti-CD3/CD28. Knock-out of PHGDH was confirmed 96 hours post-activation by flow cytometry (Figure 4A). Intriguingly, frequency of late and early apoptotic cells was increased, inversely frequency of viable cells significantly decreased, upon PHGDH knock-out as compared to control cells (Figure 4B, right panel).

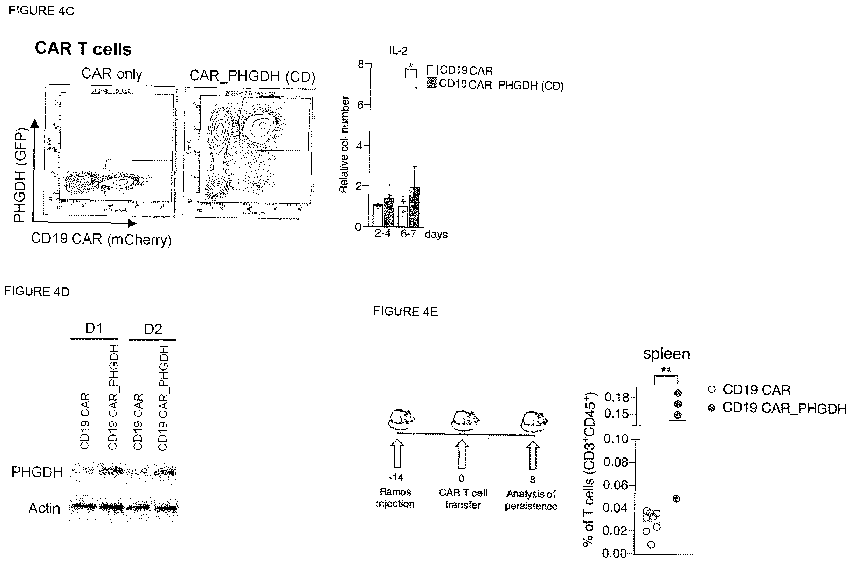

It was then tested how enforced PHGDH expression affected persistence of primary human T cells expressing a CAR. For these experiments, a CAR T cell model targeting CD19 was used. Bulk T cells were lentivirally transduced with either i) a vector encoding for a CD19 CAR and mCherry (CD19 CAR) or ii) a vector encoding for the CD19 CAR and mCherry and one encoding for the catalytic dead variant of PHGDH and GFP (CD19 CAR_PHGDH (CD)). To obtain pure CAR T cell populations cells were sorted according to mCherry and GFP expression (Figure 4C, left panel). Persistence was then assessed under non-activating conditions *in vitro* in the presence of IL-2. CD19 CAR_PHGDH (CD) cell numbers were significantly enriched at days 6-7 as compared to cells expressing the CAR only (Figure 4C, right panel).

It was then further examined whether enforced expression of PHGDH likewise affected CAR T cell persistence *in vivo.* Overexpression of PHGDH in CAR T cells was verified by Western Blot in two different donors as compared to CAR T cells (FIGURE 4D). To test the *in vivo* persistence CD19 CAR T cells or CD19 CAR_PHGDH T cells were adoptively transferred into NSG mice harboring established tumors (Figure 4E, left panel). Indeed, at 8 days post adoptive transfer frequencies of CD19 CAR_PHGDH T cells were significantly increased in spleen compared to CD19 CAR T cells (Figure 4E, right panel).

In all, these data established the importance of PHGDH in regulating human T cell persistence. Further, overexpression of PHGDH, the WT and CD variant alike, is associated with increased *in vitro* and *in vivo* persistence of cell therapeutics, in particular T cells.

### Methods

### Healthy blood donors

Blood samples were obtained from healthy female and male donors (16-65 years old) as buffy coats after written consent (Blood donation center, University Hospital Basel).

### Human cell isolation

Peripheral blood mononuclear cells (PBMC) were isolated from female and male healthy donors by standard density-gradient centrifugation protocols (#1114547, Lymphoprep Fresenius Kabi). CD8⁺ T cells were enriched by positive selection using magnetic CD8⁺ beads (#130-045-201, Miltenyi Biotec) and rested overnight in R10FBS before FACS-sorting (see below).

### Human T cell activation

Human CD8⁺ T cell activation was performed using in house generated anti-CD3/anti-CD28 coated microbeads as previously described^{1,2}. All T cell activations were performed using a 2:1 bead to cell ratio unless otherwise indicated and occurred in flat bottom 96-well plates (2.5×105 cells/well; 5×105 beads/well).

### Flow cytometry

### Cell proliferation

Cells were loaded with the cell-proliferation dye CFSE (1 µM, Molecular probes, USA) prior to activation +/- Serine and seeded in 96-well plates. CFSE dilution was analyzed 5d post-stimulation by flow cytometry. A fixable live-dead cell stain (Fixable Viability Dye, eBioscience or Zombie Aqua, Biolegend) was used to exclude dead cells prior to sample acquisition.

### Viability and Annexin V staining

Cells were harvested and washed once with PBS before staining with Aqua Zombie a fixable viability dye (BioLegend) and Annexin V (ThermoFisher) in Annexin Binding Buffer for 15 minutes at RT. Staining was quenched with Annexin binding buffer solution or FACS buffer and cells were either directly analysed by flow cytometry or washed before further intracellular staining.

### Intracellular staining

After staining the cells using Aqua Zombie and Annexin V, cells were fixed and permeabilized using intracellular Fixation and Permeabilization buffer (ThermoFisher) according to manufacturer's instructions. Then cells were stained for 45 minutes on ice with anti-PHGDH (#66350, D8F30, Cell Signaling) diluted in perm-buffer, followed by 30 minutes incubation on ice with an anti-rabbit-Alexa Fluor 594 (ThermoFisher). Cells were washed twice using perm-buffer and resuspended in FACS buffer prior sample acquisition.

All flow data were acquired on a CytoFLEX flow cytometer (Beckman Coulter) and analyzed using Flowjo 10.3 (Tree Star) unless otherwise indicated.

### FACS-sorting

For fluorescence-activated cell sorting (FACS), CD8⁺T cells were stained with anti-CD62L-APC (#21279626, LT-TD180, ImmunoTools) or anti-CD62L-PeCy7 (#304821, DREG-56, BioLegend) and anti-CD45RA-PB (#A74765, J.33, Beckman Coulter) antibodies. T_{NV}, T_{CM} and T_{EM} CD8⁺ T cells were identified as CD62L⁺ CD45RA⁺, CD62L⁺ CD45RA⁻ and CD62L⁻ CD45RA⁻populations, respectively and sorted by using a BD influx cell sorter (BD Bioscience). Cells were rested in R10FBS for a minimum of 4h at 37°C prior to experiment.

### Western blotting

Cells were washed two times with PBS and lysed in ice-cold RIPA buffer (#89900, Thermo) supplemented with protease- (#4693159001, Roche) and phosphatase inhibitors (#4906845001, Roche). Lysates were cleared by centrifugation and the protein concentration was assessed using Pierce BCA Protein Assay Kit (#23227, Thermo). Equal amounts of protein were loaded on a precast mini-PROTEAN TGX gel (#4568095, Biorad) and SDS-PAGE fractionated. After electrophoresis, total protein was assessed by the stainfree-protocol from Biorad (5min UV activation followed by imaging with a GelDoc system). Then, proteins were transferred to a nitrocellulose membrane (#1704158, Biorad) followed by blocking for 1h at RT in 5% BSA or milk in TBST. Membranes were incubated at 4°C overnight with indicated primary antibodies followed by an incubation with an horseradish peroxidase (HRP)- conjugated secondary antibodies (1h at RT). Enhanced chemiluminescence (ECL) was used for developing the Western Blot, the Geldoc system for imaging and the ImageLab software for quantification (both from Biorad).

The following primary antibodies were used: anti-PHGDH (#66350, D8F30, Cell Signaling), anti-PHGDH (ab57030, abcam, used after IP of PHGDH), anti-PSAT1 (ab154055, abcam), anti-PSPH (ab211418, abcam), anti-SLC1A4 (#8442, Cell Signaling), anti-SLC1A5 (ASCT2) (#8057, D7C12, Cell Signaling), anti-SHMT1 (#12612, Cell Signaling), anti-SHMT2 (ab155230, abcam), anti-beta-actin (#3700, 8H10D10, Cell Signaling). The following secondary antibodies were used: anti-rabbit IgG (#111- 035-144, Jackson ImmunoResearch) and antimouse IgG (#115-035-003, Jackson ImmunoResearch).

### PHGDH activity assay

Jurkat T cells expressing indicated PHGDH variants were washed once in ice cold PBS, snap frozen in liquid nitrogen and stored at -80°C until further use. At the day of the assay, cell-pellets were thawed, homogenized in lysis buffer (0.5M Tris pH 8.5, 1mM EDTA, 0.02% Triton- X, 10mM NAD) and cell debris was removed by centrifugation (16000xg for 10min at 4°C). Protein concentration of the cleared lysates was measured using Pierce BCA Protein Assay Kit (#23227, Thermo). Protein amounts were adjusted with assay buffer (50mM Tris pH 7.1, 10mM NAD) in order to have 50µg total protein in 150µl of buffer. PHGDH activity was measured in two wells for every sample, once in presence of the substrate 3-phospho-D- glycerate (3PG) (20mM, P8877, Sigma) and once in absence of it - always in a final volume of 200µl. NADH-formation by catalytic activity of PHGDH was measured with a Sinergy H1 microplate reader (Biotek) at 340nm at RT. PHGDH activity was then calculated by subtracting absorption values obtain in absence of 3PG from values obtained in presence of 3PG.

### In silico comparison of GAPDH and PHGDH

(1) Rossman fold: The Rossman folds were highlighted based on existing literature. For GAPDH, this super secondary structure is formed by residues 1-150 and 317-335 (White and Garcin, 2015). For PHGDH, the contiguous residues 102 to 288 adopt a classical Rossman fold comprising a six stranded, parallel β-sheets flanked on both sides by α-helices. Crystal structure of human 3-phosphoglycerate dehydrogenase is available online at: https://www.thesgc.org/sites/default/files/activelSee/PHGDHA_2g76_v5_351m/PHGDHA_2 g 76_v5_351m_Annotation.html Deposition Authors: Turnbull AP, Salah E, Savitsky P, Gileadi O, von Delft F, Edwards A, Arrowsmith C, Weigelt J, Sundstrom M, Oppermann (2006).
(2) The electrostatic potential: The Adaptive Poisson-Boltzmann Solver plugin³ was installed on Visual Molecular Dynamics (VMD)⁴ to compute the electrostatic potentials.
(3) The dimer interface: The GAPDH dimer interface was defined in⁵ as 'antiparallel five stranded β-sheets from adjacent subunits'. For PHGDH, a consensual definition of the dimer interface in terms of residues or secondary structures was not found in the literature. Importantly, the tetrameric form of PHGDH arranges in different ways in different organisms, i.e. they form either a type I, II or III complex⁶. The human PHGDH undergoes a complex type I structure, in which three different dimer interfaces can be defined. Here, we defined the interface as residues located within less 2.5 Å of a neighboring subunit in the I-R area as defined by⁶, and residues forming secondary structures including these interacting residues are highlighted.

### RNA immunoprecipitation sequencing (RIPseq) experiment

Immunoprecipitation: PHGDH-RNA complexes were isolated using a Magna-RIP kit (#17-701, Millipore) according to manufacturer's instructions. Briefly, around 10-15Mio sorted T_{NV} cells from 3 different human donors were activated for 36h, washed once with PBS, resuspended in 210µl of the RIP lysis buffer provided in the kit and frozen at -80°C. After thawing, lysed cells were centrifuged at 14000rpm for 10min at 4°C to clear the lysate. The resulting supernatant was distributed equally into two tubes, after removing 10µl - representing the input sample. For the IP, 4µg of anti-PHGDH antibody (#66350, D8F30, Cell Signaling) or rabbit isotype matched IgG (ab172730, abcam) was used for 50µL of magnetic protein A/G beads. The beads bound PHGDH- and isotype control antibodies were added to one of the two tubes per donor and IP was performed overnight at 4°C. After several washes, an aliquot of each sample was taken to test the efficiency of the IP by Western blotting using the mouse anti-PHGDH (ab57030, abcam) antibody. From this timepoint on, the input RNA was equally processed again. After a proteinase K digestion, RNA was isolated using TRIzol reagent (#15596026, Thermo) and the Direct-zol RNA Microprep kit (#R2062, Zymo). RNAs were eluted in 6ul of RNase-free water.

RNA integrity and quantification: RNA molecules were quality-checked on the Bioanalyzer instrument (Agilent) using the RNA 6000 Pico Chip (#5067-1513, Agilent). Amount of RNAs was quantified by fluorometry using the QuantiFluor RNA System (#E3310, Promega). Library preparation was performed, starting from 1ng total RNA, using the SMART-Seq Stranded Kit (#634444, Takara Bio). Libraries were sequenced Paired-End 51 bases (in addition: 8 bases for index 1 and 8 bases for index 2) using the NovaSeq 6000 instrument (Illumina) and the SP Flow-Cell loaded at a final concentration in Flow-Lane of 400pM and including 1% PhiX. Primary data analysis was performed with the Illumina RTA version 3.4.4. On average per sample: 38.3±18.8 millions pass-filter reads were collected on 1 SP Flow-Cell.

RNA sequencing data analysis: Read alignments were performed and count tables were obtained as described above. The data were normalized by applying the TMM method from Bioconductor edgeR package (version 3.28.1)¹. Only genes having log2 CPM counts bigger than 0 in at least 2 samples were kept for the further analyses. The principal component analysis was based on 25% of most variable genes in the dataset. The differentially expressed genes were identified using the quasi-likelihood (QL) method⁸ Implemented in edgeR package (version 3.28.1) using replicate id as covariate.

### Gene-editing of primary human T cells prior RNAseq

After the sort, T_{NV} cells were rested for 4h. Two times 2Mio cells from each donor were harvested, washed once with PBS and resuspended in 20µl electroporation buffer from the P3 Primary Cell 96-well Nucleofector Kit (V4XP-3032, Lonza). 3µM RNPs were added and electroporated with the 4D-Nucleofector (Amaxa-Lonza) program EH115. Immediately after electroporation, pre-warmed R10FBS containing IL-2 (150U/ml, Proleukin) was added to the cells and cells were incubated for 24h.

### RNA sequencing (RNAseq) of activated PHGDH-KO T_{NV} cells

Total RNA was extracted from 2Mio cells per condition from 5 human donors. RNA was extracted using the RNeasy Mini Kit (#74106, Qiagen) according to the manufacturers' protocols, including an on-column DNAse (#79254, Qiagen) treatment.

Sample preparation and sequencing: RNA integrity was checked on the TapeStation instrument (Agilent) using the RNA ScreenTape (#5067-5576, Agilent) and quantified by fluorometry using the QuantiFluor RNA System (#E3310, Promega). Library preparation was performed, starting from 200ng total RNA, using the TruSeq Stranded mRNA Library Kit (#20020595, Illumina), the TruSeq RNA UD Indexes (#20022371, Illumina) and the TruSeq RNA CD Index Plate (#20019792, Illumina). 15 cycles of PCR were performed. Resulting libraries were sequenced Paired-End 101 bases (in addition: 8 bases for index 1 and 8 bases for index 2) using the NovaSeq 6000 instrument (Illumina) and the S2 Flow-Cell loaded with a final concentration of 380pM per Flow-lane and including 1% PhiX. Primary data analysis was performed with the Illumina RTA version 3.4.4. On average per sample: 69.1±11.5 millions pass-filter reads were collected on one S2 Flow-Cell.

RNA sequencing data analysis: Reads were aligned to the human genome (UCSC version hg38 analysis set) with STAR (version 2.7.0c)⁹ with default parameters except for allowing up 10 hits to genome (outFilterMultimapNmax 10), reporting only one location for multimapping reads with equal score (outSAMmultNmax 1), and for filtering reads without evidence in spliced junction table (outFilterType "BySJout"). The output was sorted and indexed with samtools (v 1.9)¹⁰. Stand-specific coverage tracks per sample were generated by tiling the genome in 20bp windows and counting 5'end of reads per window using the function bamCount from the Bioconductor package bamsignals (v1.18.0). These window counts were exported in bigWig format using the Bioconductor package rtracklayer (v1.46.0)¹¹. Read and alignment quality was evaluated using the qQCReport function of the Bioconductor package QuasR (v 1.26.0)¹². The featureCounts function from Bioconductor package subread (v 2.0.1)¹³ was used to count the number of reads (5'ends) overlapping with the exons of each gene assuming an exon union model (with used gene model provided by ensembl v96).

### Recombinant lentivirus production

24 hours before transfection, HEK-293 cells were seeded (5×10⁶ cells/5 ml media) in a 10 cm dish. All plasmid DNA was purified using the Endotoxin-free Plasmid Midiprep Kit (Qiagen). HEK-293T cells were transfected with 9.15µg pmol psPAX2 (lentiviral packaging plasmid) and 2.77µg pMD2G (VSV-G envelope expressing plasmid) and 9.2µg of pLV-CAR:FMC63-BBZ-P2A-mCherry (CAR-mC) or pLV-CAR:FMC63-BBZ-P2A-PHGDH-P2A-mCherry (CAR-PHGDH-mC) using Lipofectamine 2000 (Invitrogen) and Optimem medium (Invitrogen, Life Technologies).

In some experiments HEK-293T cells were transfected with 7.2µg pCMVR8.74 (lentiviral packaging plasmid) and 2.8µg pCMV-VSV-G (VSV-G envelope expressing plasmid) and 6µg of pSFFV-PHGDH-IRES-GFP (encoding for either the wild type or the catalytic dead variant) using Lipofectamine 2000 (Invitrogen) and Optimem medium (Invitrogen, Life Technologies). The viral supernatant was collected 48 and 72 hours after transduction. Viral particles were concentrated using VIVASPIN 20 (Sartorius) and viral supernatants were stored at -80°C. Lentiviral particles of CAR constructs were produced as described previously.

### Protocol for Lentiviral transduction

Blood samples were obtained from healthy donors after written informed consent. Peripheral blood mononuclear cells (PBMCs) were isolated by standard density-gradient centrifugation protocols (Lymphoprep; Fresenius Kabi). CD3⁺T cells were positively selected using magnetic beads (Miltenyi Biotec). CD3⁺ T cells were plated into a 24-well cell culture plate and stimulated with anti-CD3 and anti-CD28 monoclonal antibody-coated beads (Invitrogen, Life Technologies) in a ratio of 1:1 or using ImmunoCult Humam CD3/CD28 T cell activator (Stemcell) in R10AB containing IL-2 (150 U/ml). T cells were transduced with lentiviral particles at 18 - 22 hours after activation in cell culture plates coated with Polybrene (Sigma, 6 µg/ml) followed by a spinoculation step at 1000 x g for 60 min at 30°C .

Every 2 days medium was replaced with fresh IL-2 (150 U/ml). 5 days after transduction cells were analyzed for transduction efficiency by analyzing mCherry or GFP expression by flow cytometry. Samples were acquired using CytoFLEX flow cytometer (Beckman Coulter). Data were analyzed with Flowjo^{®}_V10.5 (Tree Star, USA).

To achieve pure CAR T cell populations, cells were either sorted according to mCherry expression (CD19-CAR) or both, mCherry and GFP (CD19-CAR_PHGDH). In experiments where cells were transduced with both the pSFFV-PHGDH-IRES-GFP and the pLV-CAR:FMC63-BBZ-P2A-mCherry construct double positive cells were sorted using a BD influx cell sorter (BD Bioscience).

### Proliferation of transduced T cells

Sorted T cells expressing the CD19 CAR or CD19 CAR_PHGDH (CD) were plated into a 96-well plate in R10FBS containing IL-2 (150 U/ml) at equal numbers (2×10⁵ cells/well). Cell numbers were determined by counting the cells art indicated time points.

### Animals

C57BL/6 (CD45.1 and CD45.2 as well as Thy1.1 and Thy1.2) were purchased from Charles River Laboratories. OT-I mice were purchased from The Jackson Laboratory (Bar Harbor, ME). Mice were housed under specific pathogen-free conditions at McGill University (Canada), the Van Andel Institute (USA) and the University of Basel according to approved protocols. NOD.Cg-Prkdc<scid>Il2rg<tm1Wjl>SzJ (NSG) mice were bred and housed at specific pathogen free (SPF) conditions at the University of Basel. Experiments were performed at 6 to 20 weeks of age.

### Murine T cell isolation

Following euthanasia in a CO2 chamber, spleens were harvested and pushed through a 70µm cell strainer (#431751, Corning). Red blood cells (RBC) were then removed from the single cell suspension with RBC-lysis buffer (154mM NH4CL, 10mM KHCO3 and 0.1mM EDTA) and CD8⁺ T cells MACS-isolated with CD8a-MicroBeads (#130-117-004, Miltenyi Biotec) when indicated.

### FACS sorting of T cell subsets

Murine MACS-enriched CD8⁺ T cells were stained with the following antibodies for 30min on ice using anti-CD8a-BV510, anti-CD44-AF647 and CD62L-BV421. After two washes with FACS buffer, cells were sorted on a BD FACS-Aria into T_{NV} (CD8⁺CD62L⁺CD44⁻) and T_{EM} cells (CD8⁺CD62L⁻CD44⁺).

### Adoptive transfer experiments

Plasmid constructs containing WT PHGDH (MigR1-PHGDH-IRES-EGFP; OE-PHGDH or OE-WT), or CD PHGDH (MigR1-PHGDH-IRES-EGFP; OE-CD) and control vector (MigR1- IRES-EGFP; EV) were used to transfect 293T cells together with pCMV-VSVG (Addgene). Retrovirus-containing supernatants were cleared by 0.45µm filtration and mixed with polybrene. Naive CD8⁺ Thy1.1⁺ OT-I T cells were stimulated with plate bound anti-CD3 (2ug/ml) and anti-CD28 (1ug/ml) for one day. Activated T cells were spin transduced with the EV or OE-PHGDH/OE-WT or OE-CD retroviral particles. After 24h incubation, cell culture media containing 50U/ml IL-2 was replaced and cells expanded for additional 24h before GFP-FACS-sorting. Cells were rested for 24h and then adoptively transferred into C57BL/6 mice (5000 cells/mouse). One day post transfer, mice were intravenously infected with attenuated LmOVA. Blood was sampled at indicated timepoints after infection to analyze frequency of adoptively transferred Thy1.1⁺ GFP⁺ cells of all CD8⁺ T cells by flow cytometry using a LSR Fortessa (BD Biosciences) flow cytometer.

### In vivo infection model

For memory re-challenge experiments, mice were immunized with a sublethal dose of recombinant attenuated *Listeria monocytogenes* (Lm) expressing OVA (attLmOVA, 2×106 CFU) after adoptive transfer of EV and OE-PHGDH-CD CD8⁺ Thy1.1⁺ T cells. Animals were re-infected with a lethal dose of recombinant virulent LmOVA (virLmOVA, 1×106 CFU) 35 days after the primary infection, and blood was sampled at indicated time points. Frequency of Th1.1⁺GFP⁺CD8⁺Thy1.1+ T cells was determined by flow cytometry using a LSR Fortessa (BD Biosciences) flow cytometer.

### Anti-CD19 CAR T cell in vivo experiment

CAR-mC and CAR-PHGDH-mC T cells were generated as described above and expanded for 12 days, every 2 days medium was replaced with fresh IL-2 (150 U/ml). CAR T cells were analyzed for transduction efficiency by analyzing mCherry expression by flow cytometry. Female NSG mice (6-9 weeks) were subcutaneously injected with 5×10⁵ Ramos cells (ATCC) in 50% Matrigel (Corning, standard formulation) 10 days prior adoptive transfer. At day of adoptive transfer CAR cells were counted and cell number was adjusted to adoptively transfer 1×10⁶ CAR T cells per condition.

To evaluate CAR T cell persistence in vivo, spleens were harvested, and single-cell suspension was generated. Spleens were manually homogenized in PBS and passed through a 70-µm cell strainer (BD Biosciences). ACK (Ammonium-Chloride-Potassium) lysing buffer (BD Biosciences) was used to lyse red blood cells. To determine the number of CAR T cells single-cell suspension was analyzed by flow cytometry.

### QUANTIFICATION AND STATISTICAL ANALYSIS

All plots, curve fitting, and statistical analysis were carried out with GraphPad Prism. P values of less than 0.05 were considered statistically significant.

### References

**1)** Bantug et al., 2018 (doi: 10.1016/j.immuni.2018.02.012)
**2)** Fischer et al., 2018 (doi: 10.1002/eji.201747443)
**3)** Baker et al., 2001(doi.org/10.1073/pnas.181342398)
**4)** Humphrey et al., 1996 (doi: 10.1016/0263-7855(96)00018-5)
**5)** White and Garcin, 2015 (doi: 10.1002/wrna.1315)
**6)** Grant, 2018 (doi: 10.3389/fmolb.2018.00110)
**7)** Robinson et al., 2010 (doi: 10.1093/bioinformatics/btp616)
**8)** Lund et al., 2012 (doi: 10.1515/1544-6115.1826)
**9)** Dobin et al., 2013 (doi.org/10.1093/bioinformatics/bts635)
**10)** Li et al., 2009 (doi: 10.1093/bioinformatics/btp352)
**11)** Lawrence et al., 2009 (doi: 10.1093/bioinformatics/btp328)
**12)** Gaidatzis et al., 2015 (doi.org/10.1093/bioinformatics/btu781)
**13)** Liao et al., 2019 (doi: 10.1093/nar/gkz114)

## Claims

1. A cell genetically engineered to enforce expression of phosphoglycerate dehydrogenase (PHGDH).

2. The cell of claim 2, wherein the cell is for a cellular therapy.

3. The cell of claim 1 or 2, wherein the cell is an immune cell.

4. The cell of claim 3, wherein the cell is a T cell, optionally comprising a chimeric antigen receptor (CAR).

5. The cell of any one of claims 1 to 4 comprising an exogenous polynucleotide sequence encoding PHGDH.

6. The cell of claim 5, wherein the polynucleotide sequence comprises SEQ ID NO: 4 or a sequence having 95% identity to SEQ ID NO: 4 and wherein the sequence encodes a polypeptide having RNA binding activity, or a variant or functional fragment thereof.

7. The cell of claim 5 or 6 comprising a polynucleotide sequence encoding a CAR.

8. An expression cassette comprising a polynucleotide sequence encoding phosphoglycerate dehydrogenase (PHGDH).

9. The expression cassette of claim 8 for genetically modifying a cellular therapy.

10. The expression cassette of claim 8 or 9, wherein the polynucleotide sequence comprises SEQ ID NO: 4 or a sequence having 95% identity to SEQ ID NO: 4 and wherein the sequence encodes a polypeptide having RNA binding activity, or a variant or functional fragment thereof.

11. The expression cassette of any one of claims 8 to 10, wherein the polynucleotide sequence comprises in the following 5' to 3' order:
a) a promoter sequence, and
b) a sequence encoding PHGDH.

12. The expression cassette of any one of claims 8 to 11, further comprising a polynucleotide sequence encoding a chimeric antigen receptor (CAR).

13. A vector comprising a polynucleotide sequence encoding phosphoglycerate dehydrogenase (PHGDH), optionally wherein the polynucleotide sequence comprises SEQ ID NO: 4 or a sequence having 95% identity to SEQ ID NO: 4 and wherein the sequence encodes a polypeptide having RNA binding activity, or a variant or functional fragment thereof.

14. A vector comprising the expression cassette of any one of claims 8 to 12.

15. The vector of claim 13 or 14, wherein the vector is a lentiviral vector (LV), preferably an LV expression vector.

16. A cell comprising the expression cassette of any one of claims 8 to 12 or the vector of any one of claims 13 to 15.

17. A composition comprising the cell of any one of claims 1 to 7 or claim 16, the expression cassette of any one of claims 8 to 12 or the vector of any one of claims 13 to 15.

18. A method of treating a disease or condition in a subject in need thereof comprising administering to the subject the cell of any one of claims 1 to 7 or claim 16, the expression cassette of any one of claims 8 to 12, the vector of any one of claims 13 to 15 or the composition of claim 17.

19. The method of claim 18 or the cell, the expression cassette, the vector, or the composition for the use of claim 19, wherein the disease or condition is cancer or an infection.

20. The method of claim 18 or 19, wherein the method comprises a method of cellular therapy and wherein the cell of any one of claims 1 to 7 or claim 16 or the composition of claim 17 comprising the cell of any one of claims 1 to 7 or claim 16 is administered to the subject.

21. The cell of any one of claims 1 to 7 or claim 16, the expression cassette of any one of claims 8 to 12, the vector of any one of claims 13 to 15 or the composition of claim 17, for use in a method of treating a disease or condition in a subject.

22. A method of producing a cell genetically engineered to enforce expression of phosphoglycerate dehydrogenase (PHGDH) comprising contacting one or more cells with a vector of any one of claims 13 to 15.
